# EUROPEAN PATENT SPECIFICATION

(11) **EP 0 277 635 B1**
(45) Date of publication and mention of the grant of the patent: **05.01.1994**
(21) Application number: 88101472.4
(22) Date of filing: 02.02.1988
(51) Int. Cl.: C07C 211/02, C07C 211/13, C07C 211/38, C07C 317/32, C07C 209/08, C07C 209/24, A61K 31/13, A61K 31/135, C07C 211/57, C07C 211/43

(54) **Novel polyamine derivatives**
Polyaminderivate
Dérivés de polyamine

(30) Priority: 03.02.1987 US 10380
(43) Date of publication of application: 10.08.1988
(73) Proprietor: MERRELL DOW PHARMACEUTICALS INC., Cincinnati Ohio 45215-6300 (US)
(72) Inventor: Stemerick, David M., Cincinnati Ohio 45240 (US); Bitonti, Alan J., Maineville Ohio 45039 (US); Edwards, Michael J., Cincinnati Ohio 45240 (US); McCann, Peter P., Cincinnati Ohio 45241 (US); Sjoerdsma, Albert, Cincinnati Ohio 45243 (US)
(74) Representative: VOSSIUS & PARTNER

(56) References cited:
- EP-A- 0 162 413
- DE-A- 2 262 333
- DE-A- 2 458 222
- FR-A- 2 335 208
- US-A- 3 013 020
- CHEMICAL ABSTRACTS, vol.103, no. 16, October 1985, page 703, column 1, abstract no. 133893k, Columbus, Ohio, US; R. M. CLAY et al, "Non-cyclic reference ligands for tetraaza macrocycles. Synthesis and thermodynamic properties of a series of alpha,omega-di-N-methylated tetraaza ligands and their copper(II) complexes"
- CHEMICAL ABSTRACTS, vol. 73, no. 4, July 1970, page 462, column 1, abstract no. 20934t, Columbus, Ohio, US; A. OKUBO et al, "Stereochemical studies of metal chelates. V. Preparation and circular dichroism of trans-diacido(2,5,8,11-tetraazadodecane)cobalt(III) ion and its optically active derivatives"
- JOURNAL OF MEDICINAL CHEMISTRY, vol. 30, no. 1, 1987, pages 201-204; C. MELCHIORRE et al, "Differential Blockade of Muscarinic Receptor Subtypes by Polymethylene Tetraamines. Novel Class of Selective Antagonists of Cardiac M-2 Muscarinic Receptors.
- RECUEIL DES TRAVAUX CHIMIQUES DES PAYS-BAS, vol. 58, 1939, pages 544-549; J. VAN ALPHEN: "On aliphatic polyamines VIII".
- RECUEIL DES TRAVAUX CHIMIQUES DES PAYS-BAS, vol. 59, 1940, pages 31-40; J. VAN ALPHEN: "On aliphatic polyamines X".

## Description

In FR-A- 2 335 208 polyamine derivatives are disclosed which are antibiotically active.

This invention relates to novel polyamine derivatives, to the methods and intermediates useful for their preparation, and to use pharmaceutical compositions in treating diseases caused by infestation with a variety of parasitic protozoa.

More specifically, this invention relates to pharmaceutical compositions useful in the treatment of a variety of disease states caused by parasitic protozoa infesting warm-blooded animals, the treatment being the administration to the host suffering from these diseases of a pharmaceutical composition containing an antiprotozoal amount of a polyamine derivative of the formula

RHN(CH₂)ₙNH(CH₂)ₘNH(CH₂)ₙNHR I

or of a pharmaceutically acceptable salt thereof, wherein n is an integer 3 to 4, m is an integer 6 to 12, and R is a C₁₋₆ saturated or unsaturated hydrocarbyl radical or -(CH₂)ₓ-(Ar)-X wherein
- x: is zero, one or two,
- Ar: is phenyl or naphthyl, and
- X: is H, C₁₋₆ alkoxy, halogen, C₁₋₄ alkyl, -S(O)ₓR₁ with R₁ being C₁₋₆ alkyl,
said administration to the infected host being with or without conjunctive therapy with an ornithine or arginine decarboxylase inhibitor.

The invention also relates to the use of a polyamine derivative of the formula I wherein n is an integer of 2 to 6 and m is an integer of 3 to 12 and the remaining substititients are as defined above, for the preparation of a pharmaceutical composition useful in treating infections caused by protozoa.

In those instances wherein R is a saturated hydrocarbyl such compounds include those straight, branched or cyclized manifestations of alkyl radicals having up to six carbon atoms, with t-butyl and cyclohexyl being preferred, when R is an unsaturated hydrocarbyl moiety such moieties include those radicals having one or two double bonds and those having one triple bond which may be represented by such preferred radicals as -CH₂CH=CH₂, -CH₂CH₂CH=CH₂, -CH₂C≡CH, -CH₂CH=C=CH₂. R may also represent -(CH₂)ₓ-(Ar)-X wherein Ar is a phenyl or naphthyl moiety. In those instances defined by the moieties (CH₂)ₙ, wherein n is 3 to 4, such moieties include propylene, and butylene. be. In those instances defined by the (CH₂)ₘ moiety wherein such moieties include straight alkylene moieties having up to 12 carbon atoms, the preferred moieties contain , 6, 7, 8 or 9 carbon atoms. In those instances defined by -(CH₂)ₓ-(Ar)-X, it is preferred that x be one or two, Ar be unsubstituted phenyl or naphthyl, but when substituted it is preferred that the alkoxy radical be methoxy or ethoxy, the halo be chloro, the alkyl radical be methyl, ethyl or t-butyl and when S(O)ₙR₁, it is preferred that R₁ be methyl, ethyl or t-butyl and that n be either zero, one or two. In any particular compound defined by formula I, it is preferred that such compounds be symmetrical in their makeup. For example, it is preferred that for each individual compound each terminal R group be the same, and that each (CH₂)ₙ moiety be the same.

For ease in discussing and describing the concepts of this application it is convenient to use certain abbreviated forms for referring to either generic or types of compounds. For example, the compound 1,18-bis-[(phenyl)methyl]-1,5,14,18-tetraazaoctadecane of the structure ØCH₂NH(CH₂)₃NH(CH₂)₈NH(CH₂)₃NHCH₂Ø would, in its abbreviated version, be shown as BnNH(3)NH(8)NH(3)NHBn, (Bn being benzyl), and also may be referred to as a bis-benz-yl-3-8-3 compound, the nitrogen atoms obviously being understood. In the event unsymmetric compounds are employed they would be referred to as, e.g., N-benzyl-Nʹ-phenethyl-3-8-4.

In general, the compounds of formula I may be prepared by chemical reactions analogously known in the art, the choice of any specific route of preparation being dependent upon a variety of factors. For example, general availability and cost of the reactants, applicability of certain generalized reactions to specific compounds, presence of unsaturated hydrocarbyl moieties, and so forth, are all factors which are fully understood by those of ordinary skill in the art and all contribute to the choice of synthesis in the preparation of any specific compound embraced by formula I.

With the foregoing in mind, the following reaction schemes are illustrative of the pathways by which the compounds of this invention may be made.

### Reaction Scheme A

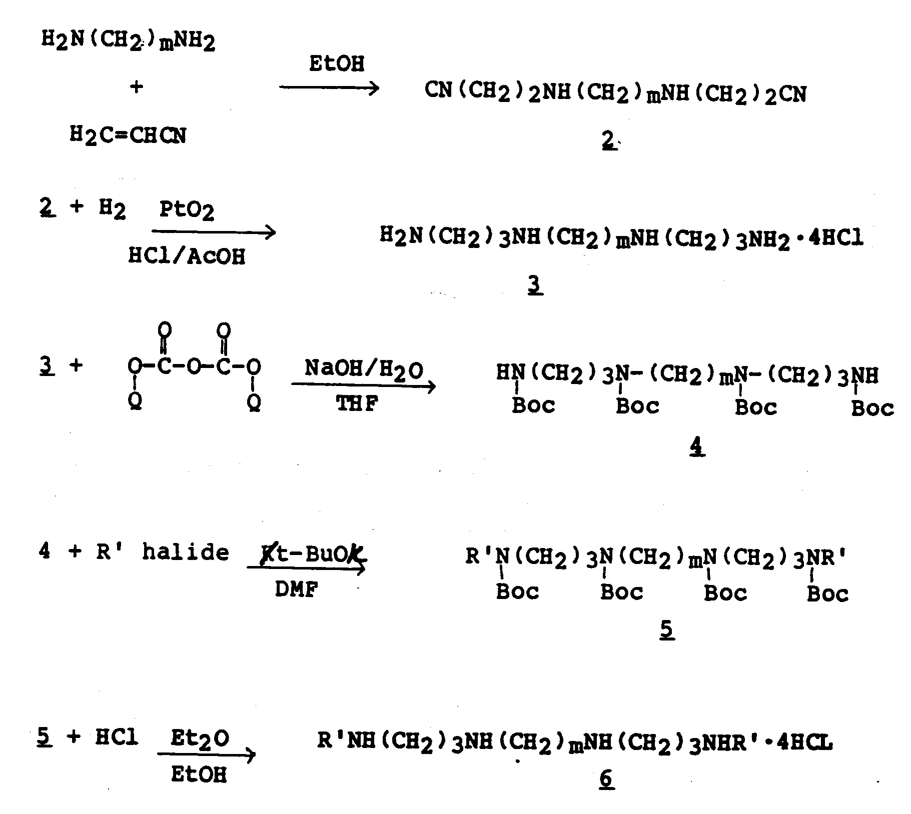

wherein R' is as defined for R in formula I except that when R is X-(Ar)-(CH₂)ₓ, x cannot be zero, Boc is the t-butoxycarbonyl protecting group, and Q is tert-butyl.

In the foregoing five step process the initial step entails a specific N-alkylation designed to produce compounds wherein n is 3 and which entails the reaction of a diamine (wherein m is as generically defined in formula I) with 2 equivalents of acrylonitrile by heating reactants, either in a suitable solvent or neat, according to standard conditions well known in the art. The resulting cyano derivatives (2) are chemically reduced by reaction with hydrogen in the presence of a catalyst (PtO₂) in a suitable solvent with 8 equivalents of hydrochloric or hydrobromic acid and to produce the resulting hydrohalic salts according to standard procedures well known in the art. Of course other reducing systems, e.g., reduction with lithium aluminum hydride, may also be utilized to produce compounds of formula 3. Following the preparation of these compounds the hydrohalic salts are neutralized with base and the nitrogen atoms are protected, preferably with di-t-butyldicarbonate according to standard operating conditions. The tetra N-protected amines (5) are alkylated with the appropriate alkyl halides (chloro or bromo) by reaction in the presence of potassium butoxide according to standard alkylation procedures well known in the art. Following alkylation the N-protective groups are removed by standard procedures, e.g., treatment with acid, preferably HCl, in the presence of a suitable solvent or solvent system, e.g., diethyloxide in ethanol, to obtain the desired products (6).

Alternatively compounds of formula 3 may be subjected to a reductive alkylation using an appropriate aldehyde (except the aldehyde cannot bear an alkylthio substituent); the reduction being effected by hydrogenation in the presence of PtO₂ according to well known procedures. This procedure does not require protection of the nitrogen atoms of the intermediates. In those instances wherein the desired final products do bear an alkylthio moiety or an unsaturated hydrocarbyl radical on their terminal nitrogen atoms, the compounds 3 (and their otherwise-prepared homologs, i.e., n is 3 or 4) may be subjected to a reductive alkylation using an appropriate aldehyde but wherein the reduction is effected with sodium cyanoborohydride according to standard techniques, this reductive alkylation again not requiring the protection of the nitrogen atoms of the intermediates.

A preferred route for the preparation of compounds of formula I wherein n is four (but which can also be applicable to those compounds wherein n is 3) and are otherwise analogous to those compounds identified as (6) in Reaction Scheme A, is the following reaction Scheme B.

### Reaction Scheme B

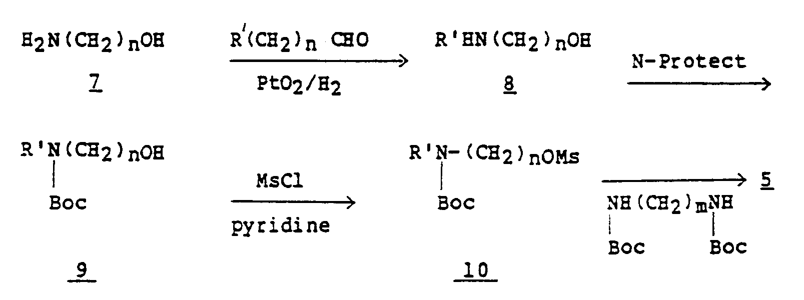

wherein n is primarily 4, but could be 3, Boc is the t-butoxycarbonyl nitrogen protecting group (which is preferred but which can be modified to any suitable N-protecting group), R' is X-(Ar)-(CH₂)ₓ with x being other than zero, and Ar' is an X-substituted alkyl or aryl (as defined in formula I), n' is zero or a positive integer, and Ms is mesyl.

This reaction is initiated by reductive alkylation techniques using an amino alcohol (7) and an appropriate aldehyde to form R' substituted amino alcohols (8) which are N-protected. The N-protected amino alcohols (9) are converted to their mesylates (10) by standard reaction conditions, e.g., reaction with mesylchloride in the presence of pyridine, preferably in the presence of a solvent such as CH₂Cl₂.

The mesylate is subjected to alkylation with an N-protected diamine (i.e., BocNH(CH₂)ₘNHBoc) using potassium t-butoxide in a solvent (DMF) using standard procedures. The so-produced tetra N-protected tetramines (5) are deprotected as in Scheme A. In essence the foregoing reductive alkylation, N-protection, mesylation, alkylation and deprotection procedures all employ techniques and reaction conditions which are well known in the art.

A preferred method for preparing compounds wherein Ar represents phenethyl (or naphthylethyl), (particularly wherein n is 3 and m is 8) is the reaction of an aroylchloride according to the method depicted in the following Reaction Scheme C.

### Reaction Scheme C

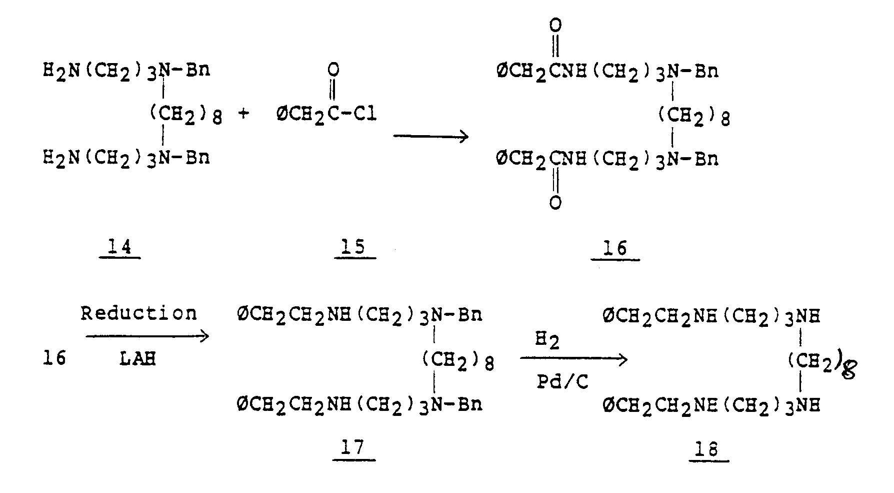

wherein Bn is benzyi, Ø is phenyl and LAH is lithium aluminum hydride. As stated above, the foregoing reaction is a preferred method for the preparation of one particular compound which entails N-alkylation of a partially protected intermediate (14) with an arylacetyl chloride (15) in the presence of triethylamine, using an inert solvent, to form an amide (16) which is chemically reduced, preferably with lithium aluminum hydride, and the resulting product (17) catalytically (E₂,Pd/C) de-benzylated to form the desired end product. These steps entail reaction techniques and procedures well known and understood in the art. Of course the same reaction scheme can be applied for the preparation of other compounds of formula I; adoption of the technique being with the usual caveats well understood by those of ordinary skill in the art.

In those instances wherein Ar represents an aromatic moiety (X-phenyl or X-naphthyl) which is attached directly to the terminal nitrogen atoms (i.e., x is zero) then such compounds may be prepared according to the general reactions of Reaction Scheme D, as follows:

### Reaction Scheme D:

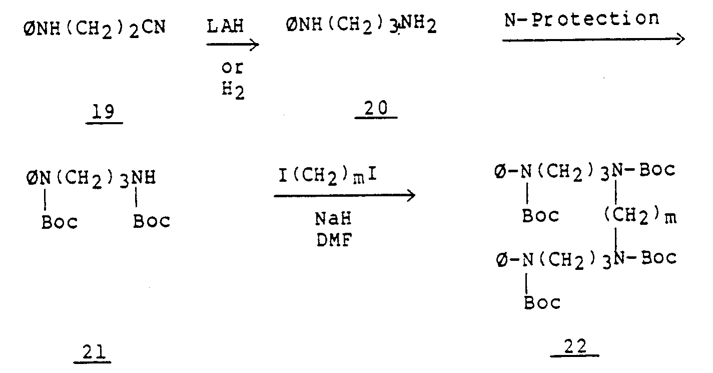

The foregoing reaction scheme depicts the preparation of compounds wherein Ar is phenyl, the first step of which is a lithium aluminum hydride reduction effected according to procedures published in the art (Bul.Soc. Chim. Fr., Part 2, 165-7 (1979)). Of course this reaction can be expanded to include naphthyl and X-substituted intermediates which will not be adversely affected by the reaction conditions. Preferably the N-protection uses the t-butoxycarbonyl protecting groups which are put on and taken off according to standard techniques already mentioned hereinabove. The N-protected compounds are alkylated by reaction with an appropriate dihalo alkane using standard and well known procedures.

In those instances wherein it is desired to prepare compounds of formula I which contain an unsaturated hydrocarbyl moiety, i.e., acetylenic, allenic, or allylic moiety-containing compounds, it is preferred to use the techniques of Reaction Scheme E, as follows:

### Reaction Scheme E:

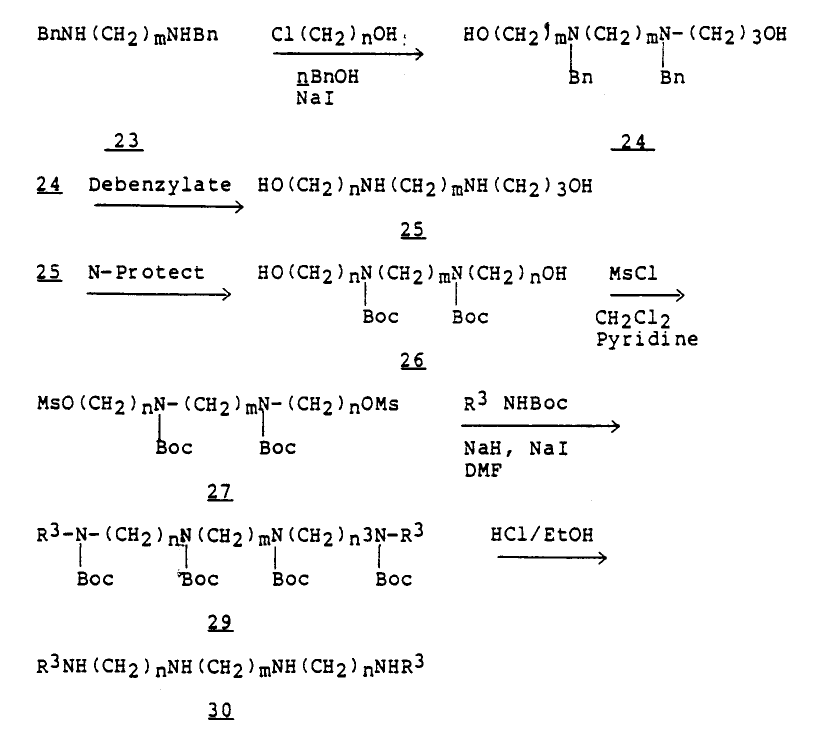

wherein R³ is an appropriate unsaturated hydrocarbyl moiety, Bn is benzyl, MsCl is methanesulfonyl chloride and Boc is the t-butoxycarbonyl protecting group.

In the foregoing reaction a dibenzylated diamine (23) is N-alkylated by a simple displacement reaction to form compounds (24) which are sequentially benzylated and N-protected. These steps are effected according to well known and standard procedures. The resulting bis(hydroxy)aminoalkanes (26) are mesylated and the mesylates (27) are alkylated with two equivalents of an N-protected amine bearing an appropriate unsaturated hydrocarbyl moiety, e.g., N-(t-butoxycarbonyl)-2,3-butadienylamine. A so-obtained tetra protected tetramine (29) is then readily de-protected to produce the desired compounds (30).

In those instances wherein it is desired to convert an alkylthio substituent to one of its higher oxidation states the alkyl thioether is treated with a peracid according to known conditions. Suitable oxidizing agents are H₂O₂ and NaIO₄ but meta-chloroperoxybenzoic acid is preferred. In effecting the oxidation to a sulfinyl derivative 1 molar equivalent (per alkylthioether moiety) is used and 2 molar equivalents of the peracid will yield the sulfonyl derivatives. The oxidations are effected at temperatures of about 0°C to room temperature in solvents which themselves are not susceptible to oxidation. Preferred solvents are CH₂Cl₂, CHCl₃, acetic acid and ethyl acetate.

### Illustrative Examples for Reaction Scheme A

### EXAMPLE 1

### 1,18-Bis[(phenyl)methyl]1,5,14,18-tetraazaoctadecane·4HCl

Step A. N,N'-Bis-[2,2'-bis(cyano)ethyl]-1,8-diaminooctane. Dissolve 28.8 gm (0.2 mol) of 1,8-diaminooctane in 250 ml of EtOH. Add 27 ml (0.41 mol) of acrylonitrile and the mixture is gently refluxed overnight. Remove the solvent at reduced pressure. Analysis showed desired material to be >95% pure.

Step B. 1,5,14,18-Tetraazaoctadecane tetrahydrochloride. Combine 50.0 gm of the product of Example 1, 2.0 gm PtO₂, 133 ml of conc. HCl and 600 ml of AcOH, treat the resulting mixture with H₂ at (45 lbs./sq.in) 3,10365 bar in a shaker flask until hydrogen is no longer taken up. Filter the resulting mixture, evaporate the solvent and triturate the product with 1 liter of EtOH. Filter and dry the product to obtain 51.6 gm of the title compound, Rf is 0.17 (silica gel plates eluted with 40% conc. NH₃/CH₃OH).

Step C. 1,5,14,18-Tetra(t-butoxycarbonyl)-1,5,14,18-tetraazaoctadecane. Treat 28.0 gm (0.069 mol) of the product of Step B with 10.99 gm (0.274 mol) of NaOH in 120 ml H₂O. When a homogenous solution is obtained add 65.7 gm (0.307 mol) of di-t-butyldicarbonate in 750 ml of THF and stir the resulting mixture for 16 hours. Separate the layers, remove and wash (2x) the aqueous layer with 500 ml CH₂Cl₂. Combine and dry (MgSO₄) the organics, filter and evaporate (in vacuo) the solvents and flash chromatograph the residue (silica gel), eluting with 25% EtOAc/hexane to yield 30.2 g of the desired product. Rf is 0.33 (silica gel plates eluted with 25% EtOAc/hexane).

Step D. 1,18-Bis[(phenyl)methyl]-1,5,14,18-tetra(t-butoxycarbonyl)-1,5,14,18-tetraazaoctadecane. Dissolve 20.0 gm (0.03 mol) of the product from Step C in 30 ml DMF and treat with 7.5 gm (0.067 mol) tBuOK and 7.96 ml (0.067 mol) BnBr, with stirring for 18 hours. Evaporate the volatiles ((0.5 mm Hg) 66,66 Pa and 45°C) and the resulting residue taken up in 1400 ml of EtOAc and water-washed (2x, 500 ml). The organic layer is then dried (MgSO₄) and the solvent is evaporated (in vacuo). Flash chromatography on silica gel eluted with 20% EtOAc/hexane yields 12.4 gm (50%) of desired product as a clear viscous oil. Rf is 0.42 (silica gel plates eluted with 25% EtOAc/hexane).

Step E. 1,18-Bis[(phenyl)methyl]-1,5,14,18-tetraazaoctadecane·4HCl. Dissolve 12.4 g (0.0147 mol) of the product of Step D in 14.7 ml of anhydrous EtOH and treat with 160 ml of 2N HCl in Et₂O with stirring overnight. Filter, wash the filter cake with Et₂O, and dry to obtain 7.2 gm of the desired compound, mp >300°C. Rf is 0.24 (from silica gel eluted with 10% conc. NH₃/CH₃OH).

### EXAMPLE 2

### 1,18-Bis(butyl)-1,5,14,18-tetraazaoctadecane·4 HCl

Step A. 1,18-Bis(butyl)-1,5,14,18-tetra(t-butoxycarbonyl)-1,5,14,18-tetraazaoctadecane. Combine 3.5 gm (0.0053 mol) of the product of Step C of Example 1, 2.7 gm (0.024 mol) of tBuOK 2.57 ml (0.024 mol) of 1-iodobutane in 10 ml of DMF and allow the mixture to stir for 18 hours. Evaporate the volatiles ((0.5 mm Hg) 66,66 Pa at 45°C), dissolve the residue in 500 ml of EtOAc, water-wash (2x with 100 ml) the organic layer and dry the organic layer over MgSO₄. Evaporate off the solvents and subject the residue to flash chromatography (silica gel eluted with 20% EtoAc/hexane) to yield 1.32 gm of the title compound. Rf is 0.36 (silica gel plates eluted with 20% EtOAc/hexane).

Step B. Dissolve 1.32 gm (0.0017 mol) of the product of Step A of this example in 1.7 ml of EtoH, treat with 17 ml of 2N HCl in Et₂O and stir the mixture overnight. Filter and wash the precipitate with Et₂O, recrystallize the washed material from isopropanol/water. Filter and dry the crystals (P₂O₅ at 79°C at (0.1 mm Hg) 13.332 bar to yield 0.62 gm of the title compound, mp >300°C. Rf is 0.47 (silica gel plates eluted with 20% conc. NH₃/CH₃OH).

### EXAMPLE 2A

### 1,18-Bis[(1-Naphthyl)methyl]-1,5,14,18-tetraazaoctadecane tetrahydrochloride hemihydrate

Step A. A solution of 1-chloromethylnaphthalene (1.7 g - 9.6 mmol) in 5 ml hexamethylphospborous triamide (HMPA) is added to a mixture of 1,5,14,18-tetra(t-butoxycarbonyl)-1,5,14,18-tetraazactadecane (2.6 g) and potassium t-butoxide (1.07 gm) in HMPA (50 ml). The mixture is heated in an 80°C oil bath for 4 hours, poured into 300 ml of water, and the aqueous mixture is extracted with ethyl acetate (2x 300 ml). The combined extracts are extracted with water (3x 300 ml) and brine (300 ml). The organic layer is dried, evaporated and the residue is chromatographed (flash-silica gel column eluting with 4 to 1 toluene/ethylacetate) to yield 800 mg of 1,18-bis-[(1-naphthyl)methyl]-1,5,14,18-tetra(t-butoxycarbonyl)1,5,14, 18-tetraazaoctadecane.

Step B. The product of Step A (800 mg) is dissolved in methanol (50 ml), excess HCl gas is added and the resulting mixture stirred overnight at ambient temperature. The mixture is filtered, the solids vacuum dried to yield the desired product of this example, mp 262-264°C.

### Illustrative Examples for Reaction Scheme B

### EXAMPLE 3

### 1,20-Bis[(phenyl)methyl]-1,6,15,20-tetraazaeicosane·4 HCl

Step A. N,N'-Bis(t-butoxycarbonyl)-1,8-octanediamine Dissolve 10.8 gm (0.075 mol) of diaminooctane in 200 ml CH₂Cl₂ and 100 ml CH₃OH, add 32.7 gm (0.156 mol) of di-t-butyldicarbonate and stir the mixture overnight. Evaporate, in vacuo, and crystallize the residue from hexane to obtain 20.2 gm of the desired compound, mp 96-97°C.

Step B. 4-[[(Phenyl)methyl]amino]-butan-1-ol. Combine 4-amino-butan-1-ol (8.9 gm - 0.1 mol), benzaldehyde (10.6 gm - 0.1 mol), EtOH (100 ml) and PtO₂ (0.3 gm), and hydrogenate the mixture at (45 lbs./sq.in) 3,10365 bar until H₂ is no longer taken up. Filter, evaporate the solvent (in vacuo) to yield 17.7 gm of the desired compound. Rf is 0.70 (eluted from silica gel with 10% conc. NH₃/CH₃OH).

Step C. 4-[N-(t-butoxycarbonyl)N-[(phenyl)methyl]-amino]butan-1-ol. Combine the butanol of Step B (17.7 g - 0.1 mol) and di-t-butyldicarbonate in 100 ml of CH₂Cl₂ and stir the mixture overnight. Evaporate off the solvents, in vacuo, and flash chromatograph the residue, eluting from silica gel with 25% EtOAc/hexane to obtain the desired compound. Rf is 0.27 (silica gel plates eluted with 20% EtOAc/hexane).

Step D. 4-[N-(t-butoxycarbonyl)-N-[(phenyl)methyl]-amino]-1-methansulfonyl butane. Cool (ice-bath) a mixture containing the product of Step C (21.8 gm - 0.078 mol), 250 ml CH₂Cl₂ and 9.7 ml pyridine (0.12 mol), add in a dropwise fashion (20 minutes) mesylchloride (6.65 ml - 0.086 mol) in 6.6 ml CH₂Cl₂ and allow the mixture to warm to room temperature, stirring the mixture for 2 hours. Pour the resulting mixture into 200 ml CH₂Cl₂, wash with 500 ml 0.5 N HCl, saturated NaHCO₃, dry over MgSO₄, evaporate (in vacuo) and flash chromatograph eluting from the silica gel with 25% EtOAc/hexane to obtain 10.7 g of desired product, Rf is 0.36 (silica gel plates eluted with 25% EtOAc/hexane).

Step E. 1,20-Bis[(phenyl)methyl]-1,6,15,20-tetra-(t-butoxycarbonyl)-1,6,15,20-tetraazaeicosane. Admix the products of Step A (5.16 gm - 0.015 mol) and of Step D of this example (10.7 g - 0.032 mol), t-BuOK (3.92 gm), NaI (0.2 gm), and 60 ml DMF and stir the mixture for 72 hours at room temperature. Evaporate off the solvent (in vacuo), take up the residue in 600 ml EtOAc and wash (2x) with 200 ml water. Dry the organic layer (MgSO₄), evaporate the solvents, and flash chromatograph the viscous residue on silica gel eluting with 20% EtOAc/hexane to obtain the desired product, Rf is 0.22 (silica gel plates eluted with 20% EtOAc/hexane).

Step F. 1,20-Bis[(phenyl)methyl]-1,6,15,20-tetraeicosane·4 HCl. Dissolve the product of Step E (4.7 gm) (0.0054 mol) in 5 ml EtOH and treat with 54 ml of 2N HCl in Et₂O, stir the mixture overnight, filter and recrystallize to so-obtained solids from isopropanol/water. Cool, filter and dry the product (over P₂O₅ at reduced pressure) to obtain the desired product, mp >300°C, Rf is 0.47 (eluted from silica gel with 20% conc. NH₃/CH₃OH).

### Illustrative Example for Reaction Scheme C

### EXAMPLE 4

### 1,18-Bis[(2-phenyl)methyl]-1,5,14,18-tetraazaoctadecane·4 HCl hemihydrate

Step A. 1,18-Bis[[(phenyl)methyl]carbonyl]-5,14-bis-[(phenyl)methyl]-1,5,14,18-tetaazaoctadecane. A solution of 5,14-Bis[(phenyl)methyl]-1,5,14,18-tetraazaoctadecane (2.2 g, 5 mmole) and triethylamine (2 g, 20 mmol) in chloroform (100 ml) was chilled in an ice bath. A solution of phenylacetyl chloride (2.3 g, 15 mmol) in chloroform (10 ml) was added dropwise. The ice bath was removed and the mixture was stirred at ambient temperature for 18 hours. The reaction mixture was extracted with aqueous sodium bicarbonate, the organic layer was dried and evaporated. The residue was chromatographed on a flash silica gel column (ethyl acetate) to give 3 g of the desired product as a thick oil.

Step B. A solution of the product of Step A in THF (150 ml) was added dropwise to a suspension of LAH (0.5g) in THF (500 ml). The mixture was stirred for 48 hours at ambient temperature. The excess reducing agent was decomposed by dropwise addition of 1 ml of water, 1 ml of 15% NaOH then 3 ml of water. The mixture was filtered and the filtrate was evaporated. The residue was taken up in ethanol (100 ml) and anhydrous HCl gas was added to convert the product, 1,18-bis[(2-phenyl)ethyl]-5,14-bis[(phenyl)methyl]-1,5,14,18-tetraazaoctadecane, to its tetrahydrochloride salt. This product in ethanol (150 ml) was hydrogenated in the presence of Pearlman's catalyst (0.3 g) at (43 psig) 42,1665 bar on a Parr hydrogenation apparatus for 24 hours. The catalyst was filtered off and the filtrate was evaporated. The residue was crystallized from 2-propanol to give the product 1,18-bis[(2-phenyl)ethyl]-1,5,14,18-tetraazaoctadecane tetrahydrochloride salt hemihydrate, mp 228-231°C.

### Alternate Reductive Alkylation Procedures

### EXAMPLE 5

### 1,14-Bis(phenyl)methyl]-1,5,10,14-tetraazatetradecane·4 HCl.

Combine spermine (2.02 gm), 2.13 ml of benzaldehyde, 40 ml of EtOH and 0.1 gm of PtO₂, treat the mixture with H₂ (45 lbs./sq.in.) 3,10365 bar until H₂ is no longer taken up. Remove the catalyst by filtration, add 100 m 1N HCl in EtOH, add water until solids dissolve, add isopropanol until solution becomes turbid. Cool and filter, and dry the resulting solid to yield 2.04 of the desired product mp >290°C. Rf is 0.50 (eluted from silica gel with 20% conc. NH₃/CH₃OH).

### EXAMPLE 6

### 1,18-Bis[(4-methylthiophenyl)methyl]-1,5,14,18-tetraazaoctadecane·4 HCl

Combine 0.81 gm of the product B of example 1, 0.05 gm Na₂CO₃, 0.25 gm NaBH₃CN and 0.53 ml of 4-methylthiobenzaldehyde in 100 ml CH₃OH and stir the mixture overnight at room temperature. Pour the reaction mixture to 300 ml CH₂Cl₂, wash with 100 ml of 1N NaOH and 100 ml of saturated NaCl, dry over MgSO₄ and evaporate in vacuo. Recrystallize the so-obtained crude product from EtOAc, cool, filter and treat with 10 ml EtOH and 30 ml of 2N HCl in Et₂O. Filter and dry the so-obtained solid to obtain 0.32 gm of the desired product. Rf is 0.58 (eluted from silica gel with 40% conc. NH₃/CH₃OH).

### Illustrative Example of Reaction Scheme D

### EXAMPLE 7

### 1,18-Bis(phenyl)-1,5,14,18-tetraazaoctadecane

Step A. N-Phenyl-N,N'-bis(t-butoxycarbonyl)propanediamine. Cool 200 ml of anhydrous Et₂O in an ice bath and add lithium aluminum hydride (8.74 gm - 0.23 mol). Add, in a dropwise fashion over 30 minutes, 3-anilinopropionitrile (14.6 gm) in 50 ml of Et₂O, remove the ice bath, and reflux the resulting mixture overnight. Sequentially add 8.7 ml of water, 1.5 g of NaOH (in 10 ml of water) and 25 ml of water. Filter the resulting ppt, rinse with 200 ml of Et₂O and remove the solvent, in vacuo, and treat the resulting N-(phenyl) propanediamine with 43.6 g of di-t-butyldicarbonate in 600 ml of CH₂Cl₂. After stirring overnight, evaporate off the solvent and subject the residue to flash chromatography from silica gel eluting with 17% EtOAc/hexane to produce the desired compound. Rf is 0.50 (eluted from silica gel with 25% EtOAc/hexane).

Step B. 1,18-Bis(phenyl)-1,5,14,18-tetra(t-butoxycarbonyl)-1,5,14,18-tetraazaoctadecane. Stir a mixture containing the product of Step A (13.0 gm), diiodooctane (3,70 gm) and 4.14 g of potassium t-butoxide in 200 ml of DMF for about 16 hours. Evaporate the solvent at 66,66 Pa (0.5 mm Hg) and 45°C, take up the residue in 800 ml of EtOAc. Wash (2x) with 300 ml of water, dry (MgSO₄) and remove the solvent in vacuo. Subject the so-obtained viscous oil to flash chromatography, eluted with 15% EtOAc from silica gel to yield 5.7 g of the desired product. Rf is 0.36 (eluted from silica gel with EtOAc/hexane.) Remove the N-boc protecting groups according to the procedure of Step E of Example 1 to produce the title compound of this example. mp 264-267°C.

### Illustrative Examples of Reaction Scheme E

### EXAMPLE 8

### 1,18-Bis(2,3-butadienyl)-1,5,14,18-tetraazaoctadecane tetrahydrochloride.

Step A. N-(t-Butoxycarbonyl)propargylamine. In a dropwise fashion, add propargylamine (25 gm) in 25 ml of CH₂Cl₂ to a stirring mixture of di-t-butyldicarbonate (99.18 gm) in 900 ml of CH₂Cl₂. After 2 hours, remove the solvent, in vacuo, to obtain 70 gm of the desired N-protected propargylamine.

Step B. N-(t-Butoxycarbonyl)-2,3-butadienylamine. Reflux a mixture containing N-(t-butoxycarbonyl)propargylamine (70 gm), 93.5 ml of 32% formaldehyde, 76.4 ml of diisopropylamine, 19.66 gm of cuprous bromide and 860 ml of p-dioxane for 12 hours. Cool and dilute the resulting mixture with 3000 ml of Et₂O, wash with 500 ml of water, 1000 ml acetic acid, 500 ml of water (2x), 200 ml sat'd. sodium chloride, dry (MgSO₄) and evaporate in vacuo. Flash chromatograph the residue eluting from silica gel with 10% Et₂O/hexane to yield 40.8 g of the desired compound. Rf is 0.31 (eluted from silica gel with 10% EtOAc/hexane).

Step C. N,N-Bis[(phenyl)methyl]-1,8-diaminooctane. Combine 14.4 gm of diaminooctane, 20.3 ml of benzaldehyde and 0.66 gm of Pt₂O in 100 ml of ethanol. Treat the resulting mixture with hydrogen at 3,10365 bar (45 lbs./sq.in.) until no further hydrogen is taken up. Filter, evaporate the solvent (in vacuo), and distill the rendered material to obtain 25.5 gm of the desired product, bp 185-190°C at 0.1 mm.

Step D. 1,18-Bis(hydroxy)-5,14-bis[(phenyl)methyl]-5,14-diazaoctadecane. Reflux a mixture containing 25.5 g of the product of Step C, 13.2 ml of 3-chloro-1-hydroxypropane, 50.4 gm of Na₂CO₃ and 1.19 gm of sodium iodide in 40 ml of n-butanol for 18 hours. Cool the mixture and pour into 700 ml of ethylacetate, wash with water, dry over MgSO₄ and remove the solvent (in vacuo) to obtain a residue which upon distillation yields 30.0 gm of the desired product, bp 250-252°C at 13,332 bar (0.1 mm. Hg).

Step E. 1,18-Bis(hydroxy)-5,14-diazaoctadecane. Hydrogenate a mixture containing 3.0 gm of the product of Step D, 30 ml of AcOH and 0.6 gm of palladium oxide at 3.10365 bar (45 lbs./sq.in.) until no further hydrogen is taken up. Filter and remove the solvent (in vacuo) to yield 1.77 gm of the desired product, Rf is 0.37 (eluted from silica gel with 10% conc. NH₃/CH₃OH).

Step F. 1,18-Bis(hydroxy)-5,14-bis-(t-butoxycarbonyl)-5,14-diazaoctadecane. Stir a mixture containing 1.77 gm of the product of Step E, 2.97 gm (0.0136 mol) of di-t-butyldicarbonate, 3 ml of triethylamine and 50 ml of CH₂Cl₂ overnight. Dilute the mixture with 200 ml of CH₂Cl₂, wash with 200 ml of 0.5N HCl, and then 100 ml of sat'd NaCl, dry (over MgSO₄) and remove the solvent (in vacuo). Flash chromatograph the residue, eluting from silica gel with 75% EtOAc to obtain the desired product, Rf 0.29, (eluted from silica gel with 75% EtOAc/hexane).

Step G. 1,18-Bis(methanesulfonyl)-5,14-bis(t-butoxycarbonyl)-5,14-diazaoctadecane. Cool to 0°C a mixture containing 3.0 gm of the product of Step F, 3.3 ml of triethylamine and 70 ml of CH₂Cl₂. In a dropwise fashion add 1.22 ml of mesylchloride in 10 ml of CH₂Cl₂, and stir the resulting mixture at 0°C for 1-1/2 hours. Pour the mixture into 100 ml of CH₂Cl₂, wash with 100 ml of 1N AcOH, 100 ml of water, 100 ml of sat'd sodium bicarbonate, dry over MgSO₄ and remove the solvent in vacuo. Flash chromatograph the residue, eluting from silica gel with 60% EtOAc/hexane to obtain 3.5 gm of the desired product. Rf is 0.39.

Step H. 1,18-Bis(2,3-butadienyl)-1,5,14,18-tetra-(t-butoxycarbonyl)-1,5,14,18-tetraazaoctadecane. Combine a mixture containing 3.5 gm of the product of Step G, 1.74 gm of sodium iodide, 0.51 gm of hexane washed sodium hydride (60% in oil) in 12 ml of DMF with 2.16 gm of N-(t-butoxycarbonyl)-2,3-butandienylamine (i.e., the product of Step B) and allow the resulting mixture to stand for 2 hours. Remove the solvent (in vacuo), add 350 ml of ethyl acetate to the residue, wash with 50 ml of water (4x), 100 ml sat'd sodium chloride and dry over MgSO₄. Remove the solvents, in vacuo, and flash chromatograph the residue from silica gel eluting with 30% EtOAc/hexane to yield 0.5 gm of the desired product, as a viscous oil. Rf is 0.39 (eluting from silica gel with 25% EtOAc/hexane).

Step I. 1,18-Bis(2,3-butadienyl)-1,5,14,18-tetraazaoctadecane·4 HCl. Dissolve 0.5 gm of the product of Step H in 2 ml of EtOH and while stirring treat the mixture with 10 ml of 2N HCl in Et₂O. Stir the resulting mixture overnight, filter and dry the solids (in vacuo) to obtain 0.22 gm of the desired product, mp 283-284°C dec.

Using the abbreviated form for naming the compounds embraced by formula I, it is to be noted that the following specific compounds are readily prepared by applying the foregoing described techniques and procedures and by applying known prior art principles to achieve the necessary modifications:

RNH(CH₂)ₙNH(CH₂)ₘNH(CH₂)ₙNHR I

wherein the R groups are the terminal R groups and the moiety between these R groups is the "polyamine moiety".

| Polyamine Moiety | Terminal R Moieties |
|---|---|
| 3-8-3 | bis-methyl |
| 3-8-3 | bis-ethyl |
| 3-8-3 | bis-propyl |
| 3-8-3 | bis-butyl |
| 3-8-3 | bis-t-butyl |
| 3-8-3 | bis-phenyl |
| 3-8-3 | bis-naphthyl |
| 3-8-3 | bis-[(phenyl)methyl] |
| 3-8-3 | bis-[(phenyl)ethyl] |
| 3-8-3 | bis-[(naphthyl)methyl] |
| 3-8-3 | bis-[(1-naphthyl)ethyl] |
| 3-8-3 | bis-(4-chlorophenyl)methyl] |
| 3-8-3 | bis-[(4-hydroxyphenyl)methyl] |
| 3-8-3 | bis-[(4-methoxyphenyl)methyl] |
| 3-8-3 | bis-[(4-methylphenyl)methyl] |
| 3-8-3 | bis-[(4-methylthio)methyl] |
| 3-8-3 | bis-[(4-methylsulfinyl)methyl] |
| 3-8-3 | bis-[(4-methylsulfonyl)methyl] |
| 3-8-3 | bis-(acetylenyl) |
| 3-8-3 | bis-(2,3-butadienyl) |
| 3-8-3 | bis-allyl |
| 3-8-3 | bis-allenyl |

as well as their, 4-8-4, analogs, and the analogs thereof wherein the polyamine moiety is as specifically mentioned in the following chart:

| Polyamine Moiety | |
|---|---|
| 3-6-3 | 4-6-4 |
| 3-7-3 | 4-7-4 |
| 3-9-3 | 4-9-4 |
| 3-10-3 | 4-10-4 |
| 3-11-3 | 4-11-4 |
| 3-12-3 | 4-12-4 |

In their end-use application, the compounds of this invention are found to be useful in treating diseases caused by protozoal infections. In this end-use application it is also to be found that the use of conjunctive tnerapy with either an ornithine decarboxylase inhibitor or an arginne decarboxylase inhibitor will aid in the efficiency of the treatment of the particular disease state being treated.

In its generic concept the compounds of this invention (I) are useful in treating diseases caused by protozoal parasites, living either intracellularly or extracellularly in a mammalian host to be treated. Inclusive of such protozoa are parasites categorized in such genera as: plasmodium (e.g., including such species as vivax, malariae, ovale, falciparum, knowlesi, berghei, vinckei, chabaudi, gallinaceum and lophurae), Leishamania (e.g., such species as donovani, tropica, braziliensis and mexicana), Babesia (e.g., such species as bovis, rodhaini and microti), Trypanosoma (of the class stercoraria) (e.g., such as cruzi, it being noted that this species is an example of a trypansome which utilizes arginine decarboxylase in its polyamine metabolic pathway and therefore conjunctive therapy would be with an ADC inhibitor), Toxoplasma (e.g., such species as gondii) and Theileria (e.g., parva). Protozoal parasites which live outside of the blood cells include Trypanosoma (of the class salivaria) (e.g., such species as rhodesiense, gambiense, brucei, evansi, equinum, equiperdum, congolense, and vivax), Trichomonas (e.g., species such as vaginalis, foetus and gallinae), Entamoeba (e.g., such as histolytica and invadens), Pnaumocystis carinii, Eimeria (e.g., tenella, necatrix and brunetti), Cryptosporidia and Giardia (e.g., lamblia).

All of the foregoing protozoa are known to infect animals and the particular diseases for which these protozoa are responsible are well known. Thus within the scope of this invention is the use of the compounds of formula I, with or without the conjunctive use of the appropriate ornithine or arginine decarboxylase inhibitors, in the treatment of the diseases caused by the foregoing protozoa. Important human disease states for which there is a need for new therapy are Amebiasis, Malaria, Leishmaniasis, Trypanosomiasis, Toxoplasmosis, as well as the so-called opportunitistic infection diseases such as Pnaumocystic carinii.

As is well known, malaria remains the world's most important infection in terms of human suffering and death. Even today, there is a desperate need for practical, effective and safe drugs to combat this protozoal infection for, despite the pronounced advances in treatment of malaria, transmission of malaria is rising, multidrug-resistant strains of Plasmodium falciparum are spreading, and the degree of resistance to drugs of this most dangerous and prevalent plasmodial species is increasing. Indeed it has been stated that over 200 million people have malaria and over one million deaths per year are associated with malaria in Africa alone, and it is known that travel to and from those endemic regions poses an expanding health problem. Thus, a particularly important aspect of this invention is the use of the compounds of this invention (I), efficiently enhanced with conjunctive therapy with an ornithine decarboxylase inhibitor, in the treatment of malaria, including, but not limited to, such species as vivax and ovale malaria, falciparum malaria, (including cerebral malaria), malariae malaria, and blackwater fever and algid malaria.

On the basis of standard laboratory procedures (both in vitro and in vivo) well known for the evaluation of compounds useful for the treatment of protozoal infections, as well as by comparisons with known anti-protozoal agents (e.g., chloroquine the compounds of formula I are effective in the treatment of the protozoal diseases at doses of about 1 to 100 mg per kilogram of body weight per day. The preferred dose is about 10 to 30 mg/kg when administered parenterally and about 3 to 5 times that when administered enterally. Preferably the compounds, in the initial phases of treatment, are administered 3 times daily for about three days, followed by continued treatment once a day until laboratory analysis shows a cure. The preferred method of administration is intramuscularly.

Suitable ornithine decarboxylase inhibitors are such compounds as α-difluoromethylornithine, and α-monofluoromethylornithine, although other well known ODC inhibitors may also be utilized. Suitable arginine decarboxylase inhibitors are the mono- and difluoromethyl arginine compounds; the ODC and ADC inhibitors being known and available to those skilled in the art. Of course it logically follows that when any particular protozoa utilizes the arginine decarboxylase enzyme in its polyamine metabolic pathway then conjunctive therapy would utilize the ADC inhibitors; similarly when the infecting protozoa utilizes ornithine decarboxylase enzyme in its polyamine metabolic pathway then conjunctive therapy would employ an ODC inhibitor. When used the ODC and ADC inhibitors would be useful at about 50 to 500 mg/kg of body weight per day, generally extrapolated to about 10-20 gms per 70 kgm patient. In the conjunctive therapy the inhibitors would be administered at the start of the treatment with the novel polyamines of formula I and would be administered so as to maintain a suitable blood level during the entire course of treatment. Depending upon the state of the patient to be treated as judged by attending diagnostician, the ODC or ADC inhibitors preferably are administered intravenously or as solutions suitable for drinking. In practice the anti-protozoal polyamine and the arginine or ornithine decarboxylase inhibitors will not be administered in one pharmaceutical preparation. Rather it is preferred to co-administer these components as separate entities. For example, it may be preferred to administer the polyamine (I) in three equal doses, while it would be preferred to administer the decarboxylase inhibitor(s) twice daily. The important aspect to the treatment is that both medicaments be used in conjunction with each other. The most effective manner in treating the diseases with the polyamine (I) is with conjunctive therapy with the appropriate decarboxylase inhibitor.

The appropriate pharmaceutical formulations for the enteral and parenteral administrations may be prepared by procedures well known in the art such as preparing sterile physiologically acceptable solutions suitable for intramuscular injections.

As is well known in the art of pharmaceutical inventions wherein generic classes of compounds are involved, certain sub-generic and certain specific compounds are more efficient in their end-use applications than other members of the generic class. In this invention, those compounds having a center alkylene chain of 6 to 9 carbon atoms are preferred, particularly those having six, seven and eight carbon atoms. Also preferred are those compounds wherein the alkylene chains which are on either side of the center alkylene chain are those having two, three or four carbon atoms, with three being most preferred. Thus the most preferred compounds are those wherein the polyamine moiety has a 3-6-3, a 3-7-3, a 3-8-3, a 3-9-3, a 4-6-4, a 4-7-4, a 4-8-4 or a 4-9-4 constitution. The preferred are the 3-6-3, the 3-7-3, the 3-8-3 and the 3-9-3, with 3-7-3 and 3-6-3 being most preferred. The preferred terminal R group is benzyl, the compound preferably being a bis-benzyl. In all instances it has been shown that the symmetrical compounds are preferred. The most preferred is a bis-benzyl-3-7-3. In those instances wherein R is an alkyl radical methyl or ethyl are preferred or a bis-dimethyl or bis-diethyl are preferred.

The most preferred ornithine decarboxylase inhibitor is α-difluoromethyl ornithine and the most preferred arginine decarboxylase inhibitor is α-difluoromethyl arginine.

## Claims (Claims for the following Contracting State(s): AT, BE, CH, DE, FR, GB, IT, LI, LU, NL, SE)

1. A compound of the general formula I
RHN(CH₂)ₙNH(CH₂)ₘNH(CH₂)ₙNHR (I)
and the pharmaceutically acceptable salts thereof wherein n is an integer 3 to 4, m is an integer 6 to 12, and R is a C₁₋₆ saturated or unsaturated hydrocarbyl radical or -(CH₂)ₓ-(Ar)-X wherein
x is zero, one or two,
A r is phenyl or naphthyl, and
X is H, C₁₋₆ alkoxy, halogen, C₁₋₄ alkyl, -S(O)ₓR₁ with R₁ being C₁₋₆ alkyl.

2. A compound of claim 1 wherein m is an integer 6 to 9.

3. A compound of claim 2 wherein n is three.

4. A compound of claim 2 wherein R is benzyl.

5. A compound of claim 2 wherein R is phenyl.

6. A compound of claim 2 wherein R is phenylethyl.

7. A compound of claim 2 wherein said compound is bis-benzyl-3-7-3.

8. A compound of claim 2 wherein said compound is bis-benzyl-3-6-3.

9. A compound of claim 2 wherein said compound is bis-benzyl-3-8-3.

10. A compound of the general formula I
RHN(CH₂)ₙNH(CH₂)ₘNH(CH₂)ₙNHR (I)
and the pharmaceutically acceptable salts thereof wherein n is an integer 3 to 4, m is an integer 6 to 12, and R is a C₁-C₆ saturated or unsaturated hydrocarbyl radical or -(CH₂)ₓ-(Ar)-X wherein
x is zero, one or two,
Ar is phenyl or naphthyl, and
X is H, C₁-C₆ alkoxy, halogen, C₁-C₄ alkyl, -S(O)ₓR₁ with R₁ being C₁-C₆ alkyl,
for use as a therapeutic agent.

11. A compound of claim 10 for use in a method for treating infections caused by protozoa.

12. A compound of claim 10 for use in a method according to claim 11 wherein the infection is caused by protozoa of the genera consisting of Plasmodium, Trypanosoma, including salivaria and stercoraria, Leishmania, Trichomonas, Babesia, Toxoplasma, and Theileria.

13. A compound of claim 10 for use in a method according to claim 12 wherein the host suffering from a protozoal infection is suffering from malaria or from Chagas' Disease.

14. A pharmaceutical composition comprising a compound of claim 10 and a pharmaceutically acceptable carrier or diluent.

15. A pharmaceutical composition according to claim 14 further comprising an ornithine decarboxylase inhibitor or an arginine decarboxylase inhibitor.

16. A pharmaceutical composition according to claim 15 comprising α-difluoromethyl ornithine or α-difluoromethyl arginine.

17. A process for preparing a compound of the general formula I
RHN(CH₂)ₙNH(CH₂)ₘNH(CH₂)ₙNHR (I)
and the pharmaceutically acceptable salts thereof wherein n is an integer 3 to 4, m is an integer 6 to 12, and R is a C₁-C₆ saturated or unsaturated hydrocarbyl radical or -(CH₂)ₓ-(Ar)-X wherein
x is zero, one or two,
Ar is phenyl or naphthyl, and
X is H, C₁-C₆ alkoxy, halogen, C₁-C₄ alkyl, -S(O)ₓR₁ with R₁ being C₁-C₆ alkyl,
comprising
a) reaction of an appropriate N-protected tetramine with a compound of the formula
R'-halide
wherein R' is as defined for R in formula I except that when R is X-(Ar)-(CH₂)ₓ, x cannot be zero, followed by deprotection of the N-terminal alkylated product;
b) reductive alkylation of a compound of the formula
H₂N(CH₂)ₙOH
with an appropriate aldehyde R'(CH₂)_{n'}CHO wherein R' is X-(Ar)-(CH₂)ₓ with x being other than zero and n' is zero or a positive integer, N-protection and mesylation of the alkylated product and reaction of the N-protected and mesylated product with an N-protected diamine of the formula
Pro-NH(CH₂)ₘNH-Pro
wherein Pro is an N-protecting group;
c) N-aroylation of a partially N-protected tetramine with an aroyl chloride followed by reduction and deprotection of the N-aroylated product;
d) for the production of a compound of the formula I wherein R is X-(Ar)-(CH₂)ₓ wherein x is zero, reaction of an N-protected compound of the formula 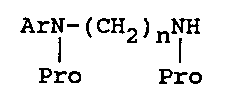 with
an appropriate dihaloalkane followed by deprotection, or
e) for the production of a compound of the formula I wherein R is an unsaturated hydrocarbyl radical, reaction of an N-protected diamine mesylate with an N-protected amine bearing an appropriate unsaturated hydrocarbyl moiety followed by deprotection.

18. Use of a compound of the general formula I
RHN(CH₂)ₙNH(CH₂)ₘNH(CH₂)ₙNHR (I)
and the pharmaceutically acceptable salts thereof wherein n is an integer 2 to 6, m is an integer 3 to 12, and R is a C₁-C₆ saturated or unsaturated hydrocarbyl radical or -(CH₂)ₓ-(Ar)-X wherein
x is zero, one or two,
Ar is phenyl or naphthyl, and
X is H, C₁-C₆ alkoxy, halogen, C₁-C₄ alkyl, -S(O)ₓR₁ with R₁ being C₁-C₆ alkyl,
for the preparation of a pharmaceutical composition useful in treating infections caused by protozoa.

## Claims (Claims for the following Contracting State(s): ES)

1. A process for preparing a compound of the general formula I
RHN(CH₂)ₙNH(CH₂)ₘNH(CH₂)ₙNHR (I)
and the pharmacerntically acceptable salts thereof wherein n is an integer 3 to 4, m is an integer 6 to 12, and R is a C₁-C₆ saturated or unsaturated hydrocarbyl radical or -(CH₂)ₓ-(Ar)-X wherein
x is zero, one or two,
Ar is phenyl or naphthyl, and
X is H, C₁-C₆ alkoxy, halogen, C₁-C₄ alkyl, -S(O)ₓR₁ with R₁ being C₁-C₆ alkyl,
a) reaction of an appropriate N-protected tetramine with a compound of the formula
R'-halide
wherein R' is as defined for R in formula I except that when R is X-(Ar)-(CH₂)ₓ, x cannot be zero, followed by deprotection of the N-terminal alkylated product;
b) reductive alkylation of a compound of the formula
H₂N(CH₂)ₙOH
with an appropriate aldehyde R'(CH₂)_{n'}CHO wherein R' is X-(Ar)-(CH₂)ₓ with x being other than zero and n' is zero or a positive integer, N-protection and mesylation of the alkylated product and reaction of the N-protected and mesylated product with an N-protected diamine of the formula
Pro-NH(CH₂)ₘNH-Pro
wherein Pro is an N-protecting group;
c) N-aroylation of a partially N-protected tetramine with an aroyl chloride followed by reduction and deprotection of the N-aroylated product;
d) for the production of a compound of the formula I wherein R is X-(Ar)-(CH₂)ₓ wherein x is zero, reaction of an N-protected compound of the formula 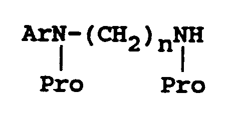 with
an appropriate dihaloalkane followed by deprotection, or
e) for the production of a compound of the formula I wherein R is an unsaturated hydrocarbyl radical, reaction of an N-protected diamine mesylate with an N-protected amine bearing an appropriate unsaturated hydrocarbyl moiety followed by deprotection.

2. A method according to claim 1 wherein in the compound prepared m is an integer 6 to 9.

3. A method according to claim 2 wherin in the compound prepared n is three.

4. A method according to claim 2 wherin in the compound prepared R is benzyl.

5. A method according to claim 2 wherin in the compound prepared R is phenyl.

6. A method according to claim 2 wherin in the compound prepared R is phenylethyl.

7. A method according to claim 2 2 wherein the compound prepared is bis-benzyl-3-7-3.

8. A method according to claim 2 wherein the compound prepared is bis-benzyl-3-6-3.

9. A method according to claim 2 wherein the compound prepared is bis-benzyl-3-8-3.

10. A method for the preparation of a pharmaceutical composition comprising combining a compound of general formula I
RHN(CH₂)ₙNH(CH₂)ₘNH(CH₂)ₙNHR (I)
and the pharmaceutically acceptable salts thereof wherein n is an integer 3 to 4, m is an integer 6 to 12, and R is a C₁-C₆ saturated or unsaturated hydrocarbyl radical or -(CH₂)ₓ-(Ar)-X wherein
x is zero, one or two,
Ar is phenyl or naphthyl, and
X is H, C₁-C₆ alkoxy, halogen, C₁-C₄ alkyl,
-S(O)ₓR₁ with R₁ being C₁-C₆ alkyl, with a pharmaceutically acceptable carrier.

11. A method according to claim 2 wherein the compound prepared is useful in treating infections caused by protozoa.

12. A method according to claim 2 wherein the compound prepared is useful in treating an infection caused by protozoa of the genera consisting of Plasmodium, Trypanosoma, including salivaria and stercoraria, Trypanosoma, including salivaria and stercoraria, Leishmania, Trichomonas, Babesia, Toxoplasma, and Theileria.

13. A method according to claim 2 wherein the compound prepared is useful in treating malaria or Chagas' Disease.

14. A method according to any one of claims 10-13 which further comprises an ornithine decarboxylase inhibitor or an arginine decarboxylase inhibitor.

15. A method according to claim 14 comprising α-difluoromethyl ornithine or α-difluoromethyl arginine.

16. A method for the preparation of a pharmaceutical composition useful in treating a protozoal infection comprising combining a compound of the general formula I
RHN(CH₂)ₙNH(CH₂)ₘNH(CH₂)ₙNHR (I)
and the pharmaceutically acceptable salts thereof wherein n is an integer 2 to 6, m is an integer 3 to 12, and R is a C₁-C₆ saturated or unsaturated hydrocarbyl radical or -(CH₂)ₓ-(Ar)-X wherein
x is zero, one or two,
Ar is phenyl or naphthyl, and
X is H, C₁-C₆ alkoxy, halogen, C₁-C₄ alkyl, -S(O)ₓR₁ with R₁ being C₁-C₆ alkyl,
with a parmaceutically acceptable carrier.

## Claims (Claims for the following Contracting State(s): GR)

1. A compound of the general formula I
RHN(CH₂)ₙNH(CH₂)ₘNH(CH₂)ₙNHR (I)
and the pharmaceutically acceptable salts thereof wherein n is an integer 3 to 4, m is an integer 6 to 12, and R is a C₁₋₆ saturated or unsaturated hydrocarbyl radical or -(CH₂)ₓ-(Ar)-X wherein
x is zero, one or two,
Ar is phenyl or naphthyl, and
X is H, C₁₋₆ alkoxy, halogen, C₁₋₄ alkyl, -S(O)ₓR₁ with R₁ being C₁₋₆ alkyl.

2. A compound of claim 1 wherein m is an integer 6 to 9.

3. A compound of claim 2 wherein n is three.

4. A compound of claim 2 wherein R is benzyl.

5. A compound of claim 2 wherein R is phenyl.

6. A compound of claim 2 wherein R is phenylethyl.

7. A compound of claim 2 wherein said compound is bis-benzyl-3-7-3.

8. A compound of claim 2 wherein said compound is bis-benzyl-3-6-3.

9. A compound of claim 2 wherein said compound is bis-benzyl-3-8-3.

10. A method for the preparation of a pharmaceutical composition comprising combining compound of the general formula I
RHN(CH₂)ₙNH(CH₂)ₘNH(CH₂)ₙNHR (I)
and the pharmaceutically acceptable salts thereof wherein n is an integer 3 to 4, m is an integer 6 to 12, and R is a C₁-C₆ saturated or unsaturated hydrocarbyl radical or -(CH₂)ₓ-(Ar)-X wherein
x is zero, one or two,
Ar is phenyl or naphthyl, and
X is H, C₁-C₆ alkoxy, halogen, C₁-C₄ alkyl, -S(O)ₓR₁ with R₁ being C₁-C₆ alkyl,
with a pharmaceutically acceptable carrier.

11. A method according to claim 10 wherein the pharmaceutical composition prepared is useful in treating infections caused by protozoa.

12. A method according to claim 10 wherein the pharmaceutical composition prepared is useful in treating an infection caused by protozoa of the genera consisting of Plasmodium, Trypanosoma, including salivaria and stercoraria, Leishmania, Trichomonas, Babesia, Toxoplasma, and Theileria.

13. A method according to claim 10 wherein the pharmaceutical composition prepared is useful in treating malaria or Chagas' Disease.

14. A method according to any one of claims 10-13 which further comprises an ornithine decarboxylase inhibitor or an arginine decarboxylase inhibitor.

15. A method according to claim 14 comprising α-difluoromethyl ornithine or α-difluoromethyl arginine.

16. A process for preparing a compound of the general formula I
RHN(CH₂)ₙNH(CH₂)ₘNH(CH₂)ₙNHR (I)
and the pharmaceutically acceptable salts thereof wherein n is an integer 3 to 4, m is an integer 6 to 12, and R is a C₁-C₆ saturated or unsaturated hydrocarbyl radical or -(CH₂)ₓ-(Ar)-X wherein
x is zero, one or two,
Ar is phenyl or naphthyl, and
X is H, C₁-C₆ alkoxy, halogen, C₁-C₄ alkyl, -S(O)ₓR₁ with R₁ being C₁-C₆ alkyl,
comprising
a) reaction of an appropriate N-protected tetramine with a compound of the formula
R'-halide
wherein R' is as defined for R in formula I except that when R is X-(Ar)-(CH₂)ₓ, x cannot be zero, followed by deprotection of the N-terminal alkylated product;
b) reductive alkylation of a compound of the formula
H₂N(CH₂)ₙOH
with an appropriate aldehyde R'(CH₂)_{n'}CHO wherein R' is X-(Ar)-(CH₂)ₓ with x being other than zero and n' is zero or a positive integer, N-protection and mesylation of the alkylated product and reaction of the N-protected and mesylated product with an N-protected diamine of the formula
Pro-NH(CH₂)ₘNH-Pro
wherein Pro is an N-protecting group;
c) N-aroylation of a partially N-protected tetramine with an aroyl chloride followed by reduction and deprotection of the N-aroylated product;
d) for the production of a compound of the formula I wherein R is X-(Ar)-(CH₂)ₓ wherein x is zero, reaction of an N-protected compound of the formula 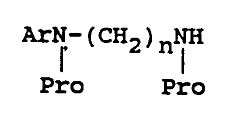 with
an appropriate dihaloalkane followed by deprotection, or
e) for the production of a compound of the formula I wherein R is an unsaturated hydrocarbyl radical, reaction of an N-protected diamine mesylate with an N-protected amine bearing an appropriate unsaturated hydrocarbyl moiety followed by deprotection.

17. A method for the preparation of a pharmaceutical composition useful in treating a protozoal infection comprising combining a compound of the general formula I
RHN(CH₂)ₙNH(CH₂)ₘNH(CH₂)ₙNHR (I)
and the pharmaceutically acceptable salts thereof wherein n is an integer 2 to 6, m is an integer 3 to 12, and R is a C₁-C₆ saturated or unsaturated hydrocarbyl radical or -(CH₂)ₓ-(Ar)-X wherein
x is zero, one or two,
Ar is phenyl or naphthyl, and
X is H, C₁-C₆ alkoxy, halogen, C₁-C₄ alkyl,
-S(O)ₓR₁ with R₁ being C₁-C₆ alkyl, with a pharameceutically acceptable carrier.

## Patentansprüche (Patentansprüche für folgende(n) Vertragsstaat(en): AT, BE, CH, DE, FR, GB, IT, LI, LU, NL, SE)

1. Verbindung der allgemeinen Formel (I)
RHN(CH₂)ₙNH(CH₂)ₘNH(CH₂)ₙNHR (I)
und die pharmazeutisch verträglichen Salze davon, wobei n eine ganze Zahl von 3 bis 4 ist, m eine ganze Zahl von 6 bis 12 ist und R ein C₁₋₆ -gesättigter oder -ungesättigter Kohlenwasserstoffrest oder ein -(CH₂)ₓ-(Ar)-X -Rest ist, wobei x die Zahl 0, 1 oder 2 ist, Ar eine Phenyl- oder Naphthylgruppe ist und X ein Wasserstoffatom, ein C₁₋₆ -Alkoxyrest, ein Halogenatom, ein C₁₋₄ -Alkyl-, oder -S(O)ₓR₁ -Rest ist, wobei R₁ ein C₁₋₆ -Alkylrest ist.

2. Verbindung nach Anspruch 1, wobei m eine ganze Zahl von 6 bis 9 ist.

3. Verbindung nach Anspruch 2, wobei n die Zahl 3 ist.

4. Verbindung nach Anspruch 2, wobei R eine Benzylgruppe ist.

5. Verbindung nach Anspruch 2, wobei R eine Phenylgruppe ist.

6. Verbindung nach Anspruch 2, wobei R eine Phenylethylgruppe ist.

7. Verbindung nach Anspruch 2, wobei die Verbindung Bis-benzyl-3-7-3 ist.

8. Verbindung nach Anspruch 2, wobei die Verbindung Bis-benzyl-3-6-3 ist.

9. Verbindung nach Anspruch 2, wobei die Verbindung Bis-benzyl-3-8-3 ist.

10. Verbindung der allgemeinen Formel (I)
RHN(CH₂)ₙNH(CH₂)ₘNH(CH₂)ₙNHR (I)
und die pharmazeutisch verträglichen Salze davon, wobei n eine ganze Zahl von 3 bis 4 ist, m eine ganze Zahl von 6 bis 12 ist und R ein C₁₋₆ -gesättigter oder -ungesättigter Kohlenwasserstoffrest oder ein -(CH₂)ₓ-(Ar)-X -Rest ist, wobei x die Zahl 0, 1 oder 2 ist, Ar eine Phenyl- oder Naphthylgruppe ist und X ein Wasserstoffatom, ein C₁₋₆ -Alkoxyrest, ein Halogenatom, ein C₁₋₄ -Alkyl-, oder -S(O)ₓR₁ -Rest ist, wobei R₁ ein C₁₋₆ -Alkylrest ist, zur Verwendung als Arzneistoff.

11. Verbindung nach Anspruch 10 zur Verwendung in einem Verfahren zur Behandlung von durch Protozoen verursachten Infektionen.

12. Verbindung nach Anspruch 10 zur Verwendung in einem Verfahren nach Anspruch 11, wobei die Infektion durch Protozoen der Gattungen Plasmodium, Trypanosoma, einschließlich salivaria und stercoraria, Leishmania, Trichomonas, Babesia, Toxoplasma oder Theileria verursacht wird.

13. Verbindung nach Anspruch 10 zur Verwendung in einem Verfahren nach Anspruch 12, wobei der an einer Protozoeninfektion leidende Wirt an Malaria oder Chagas-Krankheit leidet.

14. Arzneimittel, umfassend eine Verbindung nach Anspruch 10 und einen pharmazeutisch verträglichen Träger oder Verdünnungsmittel.

15. Arzneimittel nach Anspruch 14, weiterhin umfassend einen Ornithin-Decarboxylase-Inhibitor oder einen ArgininDecarboxylase-Inhibitor.

16. Arzneimittel nach Anspruch 15, umfassend α-Difluormethyl-Ornithin oder α-Difluormethyl-Arginin.

17. Verfahren zur Herstellung einer Verbindung der allgemeinen Formel I
RHN(CH₂)ₙNH(CH₂)ₘNH(CH₂)ₙNHR (I)
und die pharmazeutisch verträglichen Salze davon, wobei n eine ganze Zahl von 3 bis 4 ist, m eine ganze Zahl von 6 bis 12 ist und R ein c₁₋₆ -gesättigter oder -ungesättigter Kohlenwasserstoffrest oder ein -(CH₂)ₓ-(Ar)-X -Rest ist, wobei x die Zahl 0, 1 oder 2 ist, Ar eine Phenyl- oder Naphthylgruppe ist und X ein Wasserstoffatom, ein C₁₋₆ -Alkoxyrest, ein Halogenatom, ein C₁₋₄ -Alkyl-, oder -S(O)ₓR₁ -Rest ist, wobei R₁ ein C₁₋₆ -Alkylrest ist, umfassend
a) Umsetzung eines geeigneten N-geschützten Tetramins mit einer Verbindung der Formel
R'-Halogenid
wobei R' wie der Rest R in Formel I definiert ist, mit der Ausnahme, daß, wenn R ein X-(Ar)-(CH₂)ₓ -Rest ist, x nicht die Zahl 0 sein kann, gefolgt von der Entfernung der Schutzgruppe(n) vom am endständigen Stickstoff alkylierten Produkt;
b) reduktive Alkylierung einer Verbindung der Formel
H₂N(CH₂)ₙOH
mit einem geeigneten Aldehyd R'(CH₂)_{n'}CHO, wobei R' ein X-(Ar)-(CH₂)ₓ -Rest ist, wobei x nicht die Zahl 0 ist und n' die Zahl 0 oder eine positive ganze Zahl ist, Einführung einer N-Schutzgruppe und Mesylierung des alkylierten Produktes und Umsetzung des N-geschützten und mesylierten Produktes mit einem N-geschützten Diamin der Formel
Pro-NH(CH₂)ₘNH-Pro
wobei Pro eine N-Schutzgruppe ist;
c) N-Aroylierung eines teilweise N-geschützten Tetramins mit einem Aroylchlorid gefolgt von der Reduktion und Entfernung der Schutzgruppe(n) vom N-aroylierten Produkt;
d) zur Herstellung einer Verbindung der Formel I, in der R ein X-(Ar)-(CH₂)ₓ -Rest ist, wobei x die Zahl 0 ist, Umsetzung einer N-geschützten Verbindung der Formel 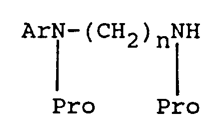 mit einem geeigneten Dihalogenalkan gefolgt von der Entfernung der Schutzgruppe(n), oder
e) zur Herstellung einer Verbindung der Formel I, in der R ein ungesättigter Kohlenwasserstoffrest ist, Umsetzung eines N-geschützten Diaminmesylats mit einem N-geschützten Amin, das eine geeignete ungesättigte Kohlenwasserstoffeinheit trägt, gefolgt von der Entfernung der Schutzgruppe(n).

18. Verwendung einer Verbindung der allgemeinen Formel I
RHN(CH₂)ₙNH(CH₂)ₘNH(CH₂)ₙNHR (I)
und der pharmazeutisch verträglichen Salze davon, wobei n eine ganze Zahl von 2 bis 6 ist, m eine ganze Zahl von 3 bis 12 ist und R ein C₁₋₆ -gesättigter oder -ungesättigter Kohlenwasserstoffrest oder ein -(CH₂)ₓ-(Ar)-X -Rest ist, wobei x die Zahl 0, 1 oder 2 ist, Ar eine Phenyl- oder Naphthylgruppe ist und X ein Wasserstoffatom, ein C₁₋₆ -Alkoxyrest, ein Halogenatom, ein C₁₋₄ -Alkyl-, oder -S(O)ₓR₁ -Rest ist, wobei R₁ ein C₁₋₆ -Alkylrestist, zur Herstellung eines Arzneimittels zur Behandlung von durch Protozoen verursachten Infektionen.

## Patentansprüche (Patentansprüche für folgende(n) Vertragsstaat(en): ES)

1. Verfahren zur Herstellung einer Verbindung der allgemeinen Formel I
RHN(CH₂)ₙNH(CH₂)ₘNH(CH₂)ₙNHR (I)
und die pharmazeutisch verträglichen Salze davon, wobei n eine ganze Zahl von 3 bis 4 ist, m eine ganze Zahl von 6 bis 12 ist und R ein C₁₋₆ -gesättigter oder -ungesättigter Kohlenwasserstoffrest oder ein -(CH₂)ₓ-(Ar)-X -Rest ist, wobei x die Zahl 0, 1 oder 2 ist, Ar eine Phenyl- oder Naphthylgruppe ist und X ein Wasserstoffatom, ein C₁₋₆ -Alkoxyrest, ein Halogenatom, ein C₁₋₄ -Alkyl-, oder -S(O)ₓR₁ -Rest ist, wobei R₁ ein C₁₋₆ -Alkylrest ist, umfassend
a) Umsetzung eines geeigneten N-geschützten Tetramins mit einer Verbindung der Formel
R'-Halogenid
wobei R' wie der Rest R in Formel I definiert ist, mit der Ausnahme, daß, wenn R ein X-(Ar)-(CH₂)ₓ -Rest ist, x nicht die Zahl 0 sein kann, gefolgt von der Entfernung der Schuzgruppe(n) vom am endständigen Stickstoff alkylierten Produkt;
b) reduktive Alkylierung einer Verbindung der Formel
H₂N(CH₂)ₙOH
mit einem geeigneten Aldehyd R'(CH₂)_{n'}CHO, wobei R' ein X-(Ar)-(CH₂)ₓ -Rest ist, wobei x nicht die Zahl 0 ist und n' die Zahl 0 oder eine positive ganze Zahl ist, Einführung einer N-Schutzgruppe und Mesylierung des alkylierten Produktes und Umsetzung des N-geschützten und mesylierten Produktes mit einem N-geschützten Diamin der Formel
Pro-NH(CH₂)ₘNH-Pro
wobei Pro eine N-Schutzgruppe ist;
c) N-Aroylierung eines teilweise N-geschützten Tetramins mit einem Aroylchlorid gefolgt von der Reduktion und Entfernung der Schutzgruppe(n) vom N-aroylierten Produkt;
d) zur Herstellung einer Verbindung der Formel I, in der R ein X-(Ar)-(CH₂)ₓ -Rest ist, wobei x die Zahl 0 ist, Umsetzung einer N-geschützten Verbindung der Formel 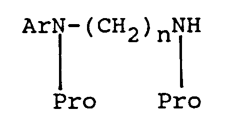 mit einem geeigneten Dihalogenalkan gefolgt von der Entfernung der Schutzgruppe(n), oder
e) zur Herstellung einer Verbindung der Formel I, in der R ein ungesättigter Kohlenwasserstoffrest ist, Umsetzung eines N-geschützten Diaminmesylats mit einem N-geschützten Amin, das eine geeignete ungesättigte Kohlenwasserstoffeinheit trägt, gefolgt von der Entfernung der Schutzgruppe(n).

2. Verfahren nach Anspruch 1, wobei m in der hergestellten Verbindung eine ganze Zahl von 6 bis 9 ist.

3. Verfahren nach Anspruch 2, wobei n in der hergestellten Verbindung die Zahl 3 ist.

4. Verfahren nach Anspruch 2, wobei Rin der hergestellten Verbindung eine Benzylgruppe ist.

5. Verfahren nach Anspruch 2, wobei R in der hergestellten Verbindung eine Phenylgruppe ist.

6. Verfahren nach Anspruch 2, wobei R in der hergestellten Verbindung eine Phenylethylgruppe ist.

7. Verfahren nach Anspruch 2, wobei die hergestellte Verbindung Bis-benzyl-3-7-3 ist.

8. Verfahren nach Anspruch 2, wobei die hergestellte Verbindung Bis-benzyl-3-6-3 ist.

9. Verfahren nach Anspruch 2, wobei die hergestellte Verbindung Bis-benzyl-3-8-3 ist.

10. Verfahren zur Herstellung eines Arzneimittels, umfassend das Vereinigen einer Verbindung der allgemeinen Formel (I)
RHN(CH₂)ₙNH(CH₂)ₘNH(CH₂)ₙNHR (I)
und der pharmazeutisch verträglichen Salze davon, wobei n eine ganze Zahl von 3 bis 4 ist, m eine ganze Zahl von 6 bis 12 ist und R ein C₁₋₆ -gesättigter oder -ungesättigter Kohlenwasserstoffrest oder ein -(CH₂)ₓ-(Ar)-X -Rest ist, wobei x die Zahl 0, 1 oder 2 ist, Ar eine Phenyl- oder Naphthylgruppe ist und X ein Wasserstoffatom, ein C₁₋₆ -Alkoxyrest, ein Halogenatom, ein C₁₋₄ -Alkyl-, oder -S(O)ₓR₁ -Rest ist, wobei R₁ ein C₁₋₆ -Alkylrest ist, mit einem pharmazeutisch verträglichen Träger.

11. Verfahren nach Anspruch 2, wobei das hergestellte Arzneimittel nützlich ist zur Verwendung in einem Verfahren zur Behandlung von durch Protozoen verursachten Infektionen.

12. Verfahren nach Anspruch 2, wobei das hergestellte Arzneimittel nützlich ist zur Behandlung einer Infektion, die durch Protozoen der Gattungen Plasmodium, Trypanosoma, einschließlich salivaria und stercoraria, Leishmania, Trichomonas, Babesia, Toxoplasma oder Theileria verursacht wird.

13. Verfahren nach Anspruch 2, wobei das hergestellte Arzneimittel nützlich ist zur Behandlung von Malaria oder Chagas-Krankneit.

14. Verfahren nach einem der Ansprüche 10 bis 13, wobei das hergestellte Arzneimittel weiterhin einen Ornithin-Decarboxylase-Inhibitor oder einen Arginin-Decarboxylase-Inhibitor umfaßt.

15. Verfahren nach Anspruch 14, wobei das hergestellte Arzneimittel α-Difluormethyl-Ornithin oder α-Difluormethyl-Arginin umfaßt.

16. Verfahren zur Herstellung eines bei der Behandlung von einer durch Protozoen verursachten Infektion nützlichen Arzneimittels, umfassend das Zusammenbringen einer Verbindung der allgemeinen Formel I
RHN(CH₂)ₙNH(CH₂)ₘNH(CH₂)ₙNHR (I)
und der pharmazeutisch verträglichen Salze davon, wobei n eine ganze Zahl von 2 bis 6 ist, m eine ganze Zahl von 3 bis 12 ist und R ein C₁₋₆ -gesättigter oder -ungesättigter Kohlenwasserstoffrest oder ein -(CH₂)ₓ-(Ar)-X -Rest ist, wobei x die Zahl 0, 1 oder 2 ist, Ar eine Phenyl- oder Naphthylgruppe ist und X ein Wasserstoffatom, ein C₁₋₆ -Alkoxyrest, ein Halogenatom, ein C₁₋₄ -Alkyl-, oder -S(O)ₓR₁ -Rest ist, wobei R₁ ein C₁₋₆ -Alkylrest ist, mit einem pharmazeutisch verträglichen Träger.

## Patentansprüche (Patentansprüche für folgende(n) Vertragsstaat(en): GR)

1. Verbindung der allgemeinen Formel (I)
RHN(CH₂)ₙNH(CH₂)ₘNH(CH₂)ₙNHR (I)
und die pharmazeutisch verträglichen Salze davon, wobei n eineganze Zahl von 3 bis 4 ist, m eine ganze Zahl von 6 bis 12 ist und R ein C₁₋₆ -gesättigter oder -ungesättigter Kohlenwasserstoffrest oder ein -(CH₂)ₓ-(Ar)-X -Rest ist, wobei x die Zahl 0, 1 oder 2 ist, Ar eine Phenyl- oder Naphthylgruppe ist und X ein Wasserstoffatom, ein C₁₋₆ -Alkoxyrest, ein Halogenatom, ein C₁₋₄ -Alkyl-, oder -S(O)ₓR₁ -Restist, wobei R₁ ein C₁₋₆ -Alkylrest ist.

2. Verbindung nach Anspruch 1, wobei m eine ganze Zahl von 6 bis 9 ist.

3. Verbindung nach Anspruch 2, wobei n die Zahl 3 ist.

4. Verbindung nach Anspruch 2, wobei R eine Benzylgruppe ist.

5. Verbindung nach Anspruch 2, wobei R eine Phenylgruppe ist.

6. Verbindung nach Anspruch 2, wobei R eine Phenylethylgruppe ist.

7. Verbindung nach Anspruch 2, wobei die Verbindung Bis-benzyl-3-7-3 ist.

8. Verbindung nach Anspruch 2, wobei die Verbindung Bis-benzyl-3-6-3 ist.

9. Verbindung nach Anspruch 2, wobei die Verbindung Bis-benzyl-3-8-3 ist.

10. Verfahren zur Herstellung eines Arzneimittels, umfassend das Vereinigen einer Verbindung der allgemeinen Formel (I)
RHN(CH₂)ₙNH(CH₂)ₘNH(CH₂)ₙNHR (I)
oder eines pharmazeutisch verträglichen Salzes davon, wobei n eine ganze Zahl von 3 bis 4 ist, m eine ganze Zahl von 6 bis 12 ist und R ein C₁₋₆ -gesättigter oder -ungesättigter Kohlenwasserstoffrest oder ein -(CH₂)ₓ-(Ar)-X- Rest ist, wobei x die Zahl 0, 1 oder 2 ist, Ar eine Phenyl- oder Naphthylgruppe ist und X ein Wasserstoffatom, ein C₁₋₆ -Alkoxyrest, ein Halogenatom, ein C₁₋₄ -Alkyl-, oder -S(O)ₓR₁ -Rest ist, wobei R₁ ein C₁₋₆ -Alkylrest ist, mit einem pharmazeutisch verträglichen Träger.

11. Verfahren nach Anspruch 10, wobei das hergestellte Arzneimittel nützlich ist zur Behandlung von durch Protozoen verursachten Infektionen.

12. Verfahren nach Anspruch 10, wobei das hergestellte Arzneimittel nützlich ist zur Behandlung einer Infektion, die durch Protozoen der Gattungen Plasmodium, Trypanosoma, einschließlich salivaria und stercoraria, Leishmania, Trichomonas, Babesia, Toxoplasma oder Theileria verursacht wird.

13. Verfahren nach Anspruch 10, wobei das hergestellte Arzneimittel nützlich ist zur Behandlung von Malaria oder Chagas-Krankneit.

14. Verfahren nach einem der Ansprüche 10 bis 13, wobei das Arzneimittel weiterhin einen Ornithin-Decarboxylase-Inhibitor oder einen Arginin-Decarboxylase-Inhibitor umfaßt.

15. Verfahren nach Anspruch 14, wobei das Arzneimittel α-Difluormethyl-Ornithin oder α-Difluormethyl-Arginin umfaßt.

16. Verfahren zur Herstellung einer Verbindung der allgemeinen Formel I
RHN(CH₂)ₙNH(CH₂)ₘNH(CH₂)ₙNHR (I)
und die pharmazeutisch verträglichen Salze davon, wobei n eine ganze Zahl von 3 bis 4 ist, m eine ganze Zahl von 6 bis 12 ist und R ein C₁₋₆ -gesättigter oder -ungesättigter Kohlenwasserstoffrest oder ein -(CH₂)ₓ-(Ar)-X -Rest ist, wobei x die Zahl 0, 1 oder 2 ist, Ar eine Phenyl- oder Naphthylgruppe ist und X ein Wasserstoffatom, ein C₁₋₆ -Alkoxyrest, ein Halogenatom, ein C₁₋₄ -Alkyl-, oder -S(O)ₓR₁ -Rest ist, wobei R₁ ein C₁₋₆ -Alkylrest ist, umfassend
a) Umsetzung eines geeigneten N-geschützten Tetramins mit einer Verbindung der Formel
R'-Halogenid
wobei R' wie der Rest R in Formel I definiert ist, mit der Ausnahme, daß, wenn R ein X-(Ar)(CH₂)ₓ -Rest ist, x nicht die Zahl 0 sein kann, gefolgt von der Entfernung der Schutzgruppe(n) vom am endständigen Stickstoff alkylierten Produkt;
b) reduktive Alkylierung einer Verbindung der Formel
H₂N(CH₂)ₙOH
mit einem geeigneten Aldehyd R'(CH₂)_{n'}CHO, wobei R' ein X-(Ar)-(CH₂)ₓ -Rest ist, wobei x nicht die Zahl 0 ist und n' die Zahl 0 oder eine positive ganze Zahl ist, Einführung einer N-Schutzgruppe und Mesylierung des alkylierten Produktes und Umsetzung des N-geschützten und mesylierten Produktes mit einem N-geschützten Diamin der Formel
Pro-NH(CH₂)ₘNH-Pro
wobei Pro eine N-Schutzgruppe ist;
c) N-Aroylierung eines teilweise N-geschützten Tetramins mit einem Aroylchlorid gefolgt von der Reduktion und Entfernung der Schutzgruppe(n) vom N-aroylierten Produkt;
d) zur Herstellung einer Verbindung der Formel I, in der R ein X-(Ar)-(CH₂)ₓ -Rest ist, wobei x die Zahl 0 ist, Umsetzung einer N-geschützten Verbindung der Formel 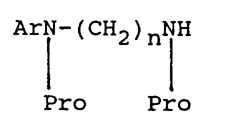 mit einem geeigneten Dihalogenalkan gefolgt von der Entfernung der Schutzgruppe(n), oder
e) zur Herstellung einer Verbindung der Formel I, in der R ein ungesättigter Kohlenwasserstoffrest ist, Umsetzung eines N-geschützten Diaminmesylats mit einem N-geschützten Amin, das eine geeignete ungesättigte Kohlenwasserstoffeinheit trägt, gefolgt von der Entfernung der Schutzgruppe(n).

17. Verfahren zur Herstellung eines bei der Behandlung von durch Protozoen verursachten Infektionen nützlichen Arzneimittels, umfassend das Zusammenbringen einer Verbindung der allgemeinen Formel I
RHN(CH₂)ₙNH(CH₂)ₘNH(CH₂)ₙNHR (I)
und der pharmazeutisch verträglichen Salze davon, wobei n eine ganze Zahl von 2 bis 6 ist, m eine ganze Zahl von 3 bis 12 ist und R ein C₁₋₆ -gesättigter oder -ungesättigter Kohlenwasserstoffrest oder ein -(CH₂)ₓ-(Ar)-X -Rest ist, wobei x die Zahl 0, 1 oder 2 ist, Ar eine Phenyl- oder Naphthylgruppe ist und X ein Wasserstoffatom, ein C₁₋₆ -Alkoxyrest, ein Halogenatom, ein C₁₋₄ -Alkyl-, oder -S(O)ₓR₁ -Rest ist, wobei R₁ ein C₁₋₆ -Alkylrest ist, mit einem pharmazeutisch verträglichen Träger.

## Revendications (Revendications pour l'(les) Etat(s) contractant(s) suivant(s): AT, BE, CH, DE, FR, GB, IT, LI, LU, NL, SE)

1. Un composé de la formule générale I
RHN (CH₂)ₙ NH (CH₂)ₘ NH (CH₂)ₙ NHR (I)
et ses sels acceptables en pharmacie dans laquelle n est un entier 3 ou 4, m est un entier de 6 à 12, et R est un radical hydrocarbyle saturé ou insaturé en C₁₋₆ ou -(CH₂)ₓ-(Ar)-X dans lequel
x est zéro, un ou deux,
Ar est un phényle ou un naphtyle, et
X est H, un alkoxy en C₁₋₆, un halogène, un alkyle en C₁₋₄, -S(O)ₓR₁ avec R₁ étant un alkyle en C₁₋₆.

2. Un composé de la revendication 1 dans laquelle m est un entier de 6 à 9.

3. Un composé de la revendication 2 dans laquelle n est trois.

4. Un composé de la revendication 2 dans laquelle R est un benzyle.

5. Un composé de la revendication 2 dans laquelle R est un phényle.

6. Un composé de la revendication 2 dans laquelle R est un phényléthyle.

7. Un composé de la revendication 2 dans laquelle ledit composé est le bis-benzyle-3-7-3.

8. Un composé de la revendication 2 dans laquelle ledit composé est le bis-benzyle-3-6-3.

9. Un composé de la revendication 2 dans laquelle ledit composé est le bis-benzyle-3-8-3.

10. Un composé de la formule générale I
RHN (CH₂)ₙ NH (CH₂)ₘ NH (CH₂)ₙ NHR (I)
et ses sels acceptables en pharmacie dans laquelle n est un entier 3 ou 4, m est un entier de 6 à 12, et R est un radical hydrocarbyle saturé ou insaturé en C₁₋₆ ou -(CH₂)ₓ-(Ar)-X dans lequel
x est zéro, un ou deux,
Ar est un phényle ou un naphtyle, et
X est H, un alkoxy en C₁₋₆, un halogène, un alkyle en C₁₋₄, -S(O)ₓR₁ avec R₁ étant un alkyle en C₁₋₆
pour être utilisé comme agent thérapeutique.

11. Un composé de la revendication 10 pour être utilisé dans une méthode de traitement des infections causées par des protozoaires.

12. Un composé de la revendication 10 pour être utilisé dans une méthode selon la revendication 11 dans laquelle l'infection est causée par des protozoaires des genres comprenant Plasmodium, Tripanosoma, incluant salivaria et stercoraria, Leishmania, Trichomonas, Babesia, Toxoplasma, et Theileria.

13. Un composé de la revendication 10 pour être utilisé dans une méthode selon la revendication 12 dans laquelle l'hôte atteint par une infection causée par un protozoaire souffre du paludisme ou de la Maladie de Chagas.

14. Une composition pharmaceutique comprenant un composé de la revendication 10 et un support acceptable en pharmacie ou un diluant.

15. Une composition pharmaceutique selon la revendication 14 comprenant en plus un inhibiteur de la décarboxylase de l'ornithine ou un inhibiteur de la décarboxylase de l'arginine.

16. Une composition pharmaceutique selon la revendication 15 comprenant l'α-difluorométhyl ornithine ou l'α-difluorométhyl arginine.

17. Un procédé pour la préparation d'un composé de la formule générale I
RHN (CH₂)ₙ NH (CH₂)ₘ NH (CH₂)ₙ NHR (I)
et ses sels acceptables en pharmacie dans laquelle n est un entier 3 ou 4, m est un entier de 6 à 12, et R est un radical hydrocarbyle saturé ou insaturé en C₁₋₆ ou -(CH₂)ₓ-(Ar)-X dans lequel
x est zéro, un ou deux,
Ar est un phényle ou un naphtyle, et
X est H, un alkoxy en C₁₋₆, un halogène, un alkyle en C₁₋₄, -S(O)ₓR₁ avec R₁ étant un alkyle en C₁₋₆,
comprenant
a) la réaction d'une tétramine avec N protégé, appropriée, avec un composé de la formule
R'-halogène
dans lequel R' est comme défini pour R dans la formule I sauf que lorsque R est X-(Ar)-(CH₂)ₓ, x ne peut pas être zéro, suivie par la déprotection de l'N terminal du produit alkylé;
b) l'alkylation réductive d'un composé de la formule
H₂N(CH₂)ₙOH
avec une aldéhyde R'(CH₂)_{n'}CHO dans laquelle R' est X-(Ar)-(CH₂)ₓ avec x étant autre que zéro et n' est zéro ou un entier positif, la protection de l'N et la mésylation du produit alkylé et la réaction du produit N-protégé et mésylé avec une diamine N-protégée de la formule
Pro-NH-(CH₂)ₘNH-Pro
dans laquelle Pro est un groupe protecteur de l'N;
c) la N-aroylation d'une tétramine partiellement N-protégée avec un chlorure d'aroyle suivie par la réduction et la déprotection du produit N-aroylé;
d) pour la production d'un composé de formule I dans lequel R est X-(Ar)-(CH₂)ₓ dans lequel x est zéro, la réaction d'un composé N-protégé de la formule 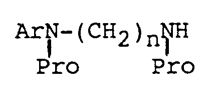 avec
un dihaloalcane suivi par une déprotection, ou
e) pour la production d'un composé de la formule I dans laquelle R est un radical hydrocarbyle insaturé, la réaction d'un mésylate diaminé N-protégé avec une amine N-protégée portant un reste hydrocarbyle insaturé, approprié, suivie par une déprotection.

18. Utilisation d'un composé de la formule générale I
RHN (CH₂)ₙ NH (CH₂)ₘ NH (CH₂)ₙ NHR (I)
et ses sels acceptables en pharmacie dans laquelle n est un entier 2 ou 6, m est un entier de 3 à 12, et R est un radical hydrocarbyle saturé ou insaturé en C₁₋₆ ou -(CH₂)ₓ-(Ar)-X dans lequel
x est zéro, un ou deux,
Ar est un pnényle ou un naphtyle, et
X est H, un alkoxy en C₁₋₆, un halogène, un alkyle en C₁₋₄, -S(O)ₓR₁ avec R₁ étant un alkyle en C₁₋₆,
pour la préparation d'une composition pharmaceutique utile dans le traitement des infections causées par des protozoaires.

## Revendications (Revendications pour l'(les) Etat(s) contractant(s) suivant(s): ES)

1. Un procédé pour la préparation d'un composé de la formule générale I
RHN (CH₂)ₙ NH (CH₂)ₘ NH (CH₂)ₙ NHR (I)
et ses sels acceptables en pharmacie dans laquelle n est un entier 3 ou 4, m est un entier de 6 à 12, et R est un radical hydrocarbyle saturé ou insaturé en C₁₋₆ ou -(CH₂)ₓ-(Ar)-X dans lequel
x est zéro, un ou deux,
Ar est un phényle ou un napntyle, et
X est H, un alkoxy en C₁₋₆, un halogène, un alkyle en C₁₋₄, -S(O)ₓR₁ avec R₁ étant un alkyle en C₁₋₆,
comprenant
a) la réaction d'une tétramine avec N protégé, appropriée, avec un composé de la formule
R'-halogène
dans lequel R' est comme défini pour R dans la formule I sauf que lorsque R est X-(Ar)-(CH₂)ₓ, x ne peut pas être zéro, suivie par la déprotection de l'N terminal du produit alkylé;
b) l'alkylation réductive d'un composé de la formule
H₂N(CH₂)ₙOH
avec une aldéhyde R'(CH₂)_{n'}CHO, appropriée, dans laquelle R' est X-(Ar)-(CH₂)ₓ avec x étant autre que zéro et n' est zéro ou un entier positif, la protection de l'N et la mésylation du produit alkylé et la réaction du produit N-protégé et mésylé avec une diamine N-protégée de la formule
Pro-NH-(CH₂)ₘNH-Pro
dans laquelle Pro est un groupe protecteur de l'N;
c) la N-aroylation d'une tétramine partiellement N-protégée avec un chlorure d'aroyle suivie par la réduction et la déprotection du produit N-aroylé;
d) pour la production d'un composé de formule I dans lequel R est X-(Ar)-(CH₂)ₓ dans lequel x est zéro, la réaction d'un composé N-protégé de la formule 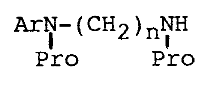 avec
un dihaloalcane approprié suivi par une déprotection, ou
e) pour la production d'un composé de la formule I dans laquelle R est un radical hydrocarbyle insaturé, la réaction d'un mésylate diaminé N-protégé avec une amine N-protégée portant un reste hydrocarbyle insaturé, approprié, suivie par une déprotection.

2. Une méthode selon la revendication 1 dans laquelle, dans le composé préparé, m est un entier de 6 à 9.

3. Une méthode selon la revendication 2 dans laquelle, dans le composé préparé, n est trois.

4. Une méthode selon la revendication 2 dans laquelle, dans le composé préparé, R est benzyle.

5. Une méthode selon la revendication 2 dans laquelle, dans le composé préparé, R est phényle.

6. Une méthode selon la revendication 2 dans laquelle, dans le composé préparé, R est phényléthyle.

7. Une méthode selon la revendication 2 dans laquelle le composé préparé est le bis-benzyle-3-7-3.

8. Une méthode selon la revendication 2 dans laquelle le composé préparé est le bis-benzyle-3-6-3.

9. Une méthode selon la revendication 2 dans laquelle le composé préparé est le bis-benzyle-3-8-3.

10. Une méthode pour la préparation d'une composition pharmaceutique comprenant l'incorporation d'un composé de la formule générale I
RHN (CH₂)ₙ NH (CH₂)ₘ NH (CH₂)ₙ NHR (I)
et ses sels acceptables en pharmacie dans laquelle n est un entier 3 ou 4, m est un entier de 6 à 12, et R est un radical hydrocarbyle saturé ou insaturé en C₁₋₆ ou -(CH₂)ₓ-(Ar)-X dans lequel
x est zéro, un ou deux,
Ar est un phényle ou un naphtyle, et
X est H, un alkoxy en C₁₋₆, un halogène, un alkyle en C₁₋₄, -S(O)ₓR₁ avec R₁ étant un alkyle en C₁₋₆,
avec un support acceptable en pharmacie.

11. Une méthode selon la revendication 2 dans laquelle le composé préparé est utile dans le traitement des infections causées par des protozoaires.

12. Une méthode selon la revendication 2 dans laquelle le composé préparé est utile dans le traitement d'une infection causée par des protozoaires des genres comprenant Plasmodium, Tripanosoma, incluant salivaria et stercoraria, Leishmania, Trichomonas, Babesia, Toxoplasma, et Theileria.

13. Une méthode selon la revendication 2 dans laquelle le composé préparé est utile dans le traitement du paludisme ou de la Maladie de Chagas.

14. Une méthode selon l'une quelconque des revendications 10-13, dans laquelle la composition préparée comprend en plus un inhibiteur de la décarboxylase de l'ornithine ou un inhibiteur de la décarboxylase de l'arginine.

15. Une méthode selon la revendication 14 dans laquelle la composition préparée comprend l'α-difluorométhyl ornithine ou l'α-difluorométhyl arginine.

16. Une méthode pour la préparation d'une composition pharmaceutique, utile dans le traitement d'une infection causée par un protozoaire, comprenant l'incorporation d'un composé de la formule générale I
RHN (CH₂)ₙ NH (CH₂)ₘ NH (CH₂)ₙ NHR (I)
et ses sels acceptables en pharmacie dans laquelle n est un entier 2 ou 6, m est un entier de 3 à 12, et R est un radical hydrocarbyle saturé ou insaturé en C₁₋₆ ou -(CH₂)ₓ-(Ar)-X dans lequel
x est zéro, un ou deux,
Ar est un phényle ou un naphtyle, et
X est H, un alkoxy en C₁₋₆, un halogène, un alkyle en C₁₋₄, -S(O)ₓR₁ avec R₁ étant un alkyle en C₁₋₆, avec un support acceptable en pharmacie.

## Revendications (Revendications pour l'(les) Etat(s) contractant(s) suivant(s): GR)

1. Un composé de la formule générale I
RHN (CH₂)ₙ NH (CH₂)ₘ NH (CH₂)ₙ NHR (I)
et ses sels acceptables en pharmacie dans laquelle n est un entier 3 ou 4, m est un entier de 6 à 12, et R est un radical hydrocarbyle saturé ou insaturé en C₁₋₆ ou -(CH₂)ₓ-(Ar)-X dans lequel
x est zéro, un ou deux,
Ar est un phényle ou un naphtyle, et
X est H, un alkoxy en C₁₋₆, un halogène, un alkyle en C₁₋₄, -S(O)ₓR₁ avec R₁ étant un alkyle en C₁₋₆.

2. Un composé de la revendication 1 dans laquelle m est un entier de 6 à 9.

3. Un composé de la revendication 2 dans laquelle n est trois.

4. Un composé de la revendication 2 dans laquelle R est un benzyle.

5. Un composé de la revendication 2 dans laquelle R est un phényle.

6. Un composé de la revendication 2 dans laquelle R est un phényléthyle.

7. Un composé de la revendication 2 dans laquelle ledit composé est le bis-benzyle-3-7-3.

8. Un composé de la revendication 2 dans laquelle ledit composé est le bis-benzyle-3-6-3.

9. Un composé de la revendication 2 dans laquelle ledit composé est le bis-benzyle-3-8-3.

10. Une méthode pour la préparation d'une composition phamaceutique comprenant l'incorporation d'un composé de la formule générale I
RHN (CH₂)ₙ NH (CH₂)ₘ NH (CH₂)ₙ NHR (I)
et ses sels acceptables en pharmacie dans laquelle n est un entier 3 ou 4, m est un entier de 6 à 12, et R est un radical hydrocarbyle saturé ou insaturé en C₁₋₆ ou -(CH₂)ₓ-(Ar)-X dans lequel
x est zéro, un ou deux,
Ar est un phényle ou un naphtyle, et
X est H, un alkoxy en C₁₋₆, un halogène, un alkyle en C₁₋₄, -S(O)ₓR₁ avec R₁ étant un alkyle en C₁₋₆,
avec un support acceptable en pharmacie.

11. Une méthode selon la revendication 10 dans laquelle la composition pharmaceutique préparée est utile dans le traitement des infections causées par des protozoaires.

12. Une méthode selon la revendication 10 dans laquelle la composition pharmaceutique préparée est utile dans le traitement d'une infection causée par des protozoaires des genres comprenant Plasmodium, Tripanosoma, incluant salivaria et stercoraria, Leishmania, Trichomonas, Babesia, Toxoplasma, et Theileria.

13. Une méthode selon la revendication 10 dans laquelle la composition pharmaceutique préparée est utile dans le traitement du paludisme ou de la Maladie de Chagas.

14. Une méthode selon l'une quelconque des revendications 10-13, dans laquelle la composition comprend en plus un inhibiteur de la décarboxylase de l'ornithine ou un inhibiteur de la décarboxylase de l'arginine.

15. Une méthode selon la revendication 14 dans laquelle la composition comprend l'α-difluorométhyl ornithine ou l'α-difluorométhyl arginine.

16. Un procédé pour la préparation d'un composé de la formule générale I
RHN (CH₂)ₙ NH (CH₂)ₘ NH (CH₂)ₙ NHR (I)
et ses sels acceptables en pharmacie dans laquelle n est un entier 3 ou 4, m est un entier de 6 à 12, et R est un radical hydrocarbyle saturé ou insaturé en C₁₋₆ ou -(CH₂)ₓ-(Ar)-X dans lequel
x est zéro, un ou deux,
Ar est un phényle ou un naphtyle, et
X est H, un alkoxy en C₁₋₆, un halogène, un alkyle en C₁₋₄, -S(O)ₓR₁ avec R₁ étant un alkyle en C₁₋₆_{,}
comprenant
a) la réaction d'une tétramine avec N protégé, appropriée, avec un composé de la formule
R'-halogène
dans lequel R' est comme défini pour R dans la formule I sauf que lorsque R est X-(Ar)-(CH₂)ₓ, x ne peut pas être zéro, suivie par la déprotection de l'N terminal du produit alkylé;
b) l'alkylation réductive d'un composé de la formule
H₂N(CH₂)ₙOH
avec une aldéhyde R'(CH₂)_{n'}CHO dans laquelle R' est X-(Ar)-(CH₂)ₓ avec x étant autre que zéro et n' est zéro ou un entier positif, la protection de l'N et la mésylation du produit alkylé et la réaction du produit N-protégé et mésylé avec une diamine N-protégée de la formule
Pro-NH-(CH₂)ₘNH-Pro
dans laquelle Pro est un groupe protecteur de l'N;
c) la N-aroylation d'une tétramine partiellement N-protégée avec un chlorure d'aroyle suivie par la réduction et la déprotection du produit N-aroylé;
d) pour la production d'un composé de formule I dans lequel R est X-(Ar)-(CH₂)ₓ dans lequel x est zéro, la réaction d'un composé N-protégé de la formule 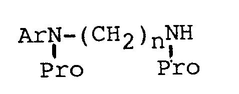 avec
un dihaloalcane suivi par une déprotection, ou
e) pour la production d'un composé de la formule I dans laquelle R est un radical hydrocarbyle insaturé, la réaction d'un mésylate diaminé N-protégé avec une amine N-protégée portant un reste hydrocarbyle insaturé, approprié, suivie par une déprotection.

17. Une méthode pour la préparation d'une composition pharmaceutique utile pour le traitement d'une infection causée par un protozoaire comprenant l'incorporation d'un composé de la formule générale I
RHN (CH₂)ₙ NH (CH₂)ₘ NH (CH₂)ₙ NHR (I)
et ses sels acceptables en pharmacie dans laquelle n est un entier 3 ou 4, m est un entier de 6 à 12, et R est un radical hydrocarbyle saturé ou insaturé en C₁₋₆ ou -(CH₂)ₓ-(Ar)-X dans lequel
x est zéro, un ou deux,
Ar est un phényle ou un naphtyle, et
X est H, un alkoxy en C₁₋₆, un halogène, un alkyle en C₁₋₄, -S(O)ₓR₁ avec R₁ étant un alkyle en C₁₋₆, avec un support acceptable en pharmacie.
